# EUROPEAN PATENT APPLICATION

(11) **EP 4 299 561 A1**
(43) Date of publication of application: **03.01.2024**
(21) Application number: 22758975.1
(22) Date of filing: 25.02.2022
(51) Int. Cl.: C07D 213/72, C07D 213/60, C07D 213/00, A61K 31/44, A61K 31/435, A61P 9/04, A61P 9/10

(54) **SUBSTITUTED PYRIDINE-2,4-DIONE DERIVATIVES**

(30) Priority: 25.02.2021 CN 202110214692; 27.01.2022 CN 202210103134; 18.02.2022 CN 202210153298
(71) Applicant: Soter Biopharma Pte. Ltd., Singapore 179803 (SG)
(72) Inventor: YAN, Xiaobing, Shanghai 200131 (CN); LAI, Wei, Shanghai 200131 (CN); DING, Charles Z., Shanghai 200131 (CN); CHEN, Shuhui, Shanghai 200131 (CN)
(74) Representative: Maiwald GmbH
(86) International application number: PCT/CN2022/077962
(87) International publication number: WO 2022/179611

(57) **Abstract**

The present invention relates to a series of substituted pyridine-2,4-dione derivatives and preparation methods therefor, and in particular, to a compound represented by formula (I) and a pharmaceutically acceptable salt thereof.

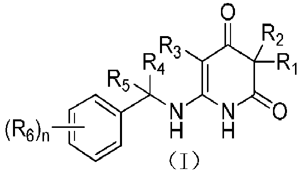

## Description

The present application claims the right of the following priorities: CN202110214692.X, February 25, 2021;
CN202210103134.0, January 27, 2022;
CN202210153298.4, February 18, 2022.

### TECHNICAL FIELD

The present disclosure relates to a series of substituted pyridine-2,4-dione derivatives and preparation methods therefor, specifically to a compound of formula (I) and a pharmaceutically acceptable salt thereof.

### BACKGROUND

Hypertrophic cardiomyopathy (HCM) is a myocardial disease characterized by myocardial hypertrophy, often invading the interventricular septum, resulting in a reduced ventricular cavity. It obstructs the filling of blood in the left ventricle and leads to decreased compliance during left ventricular diastole. HCM can be classified into obstructive and non-obstructive types based on the presence or absence of obstruction in the left ventricular outflow tract, which may be related to genetic factors. The global incidence of HCM is approximately 1/500. The clinical manifestations of HCM are diverse, ranging from asymptomatic to palpitations, exertional dyspnea, precordial pain, fatigue, syncope, and even sudden death, and left heart failure in the late stage.

Currently, the treatment medications for HCM are limited. Symptoms are mainly managed through beta-blockers or calcium channel blockers, which cannot target the cause, slow the progression of myocardial hypertrophy, or improve the prognosis. Thus, the overall therapeutic effect is limited.

Myosin and actin are the material basis for myocardial contraction. Myosin cross-bridges periodically combine with and dissociate from actin, driving myofilaments to slide, which leads to myocardial contraction. Myosin has ATPase activity, providing power for myocardial contraction through the hydrolysis of ATP. Mutations in myosin can result in prolonged binding times between myosin and actin, causing excessive contraction and impaired relaxation of the left ventricular myocardium, which leads to left ventricular myocardial hypertrophy and fibrosis, triggering HCM. MYK-461 is an allosteric regulator of myocardial myosin, which slows down phosphate hydrolysis and reduces the binding time between myosin and actin, exerting a negative inotropic effect, and alleviates pathological changes such as myocardial hypertrophy caused by excessive contraction of the left ventricular myocardium. However, its elimination from the body is slow, leading to a prolonged retention time of the medicament in the body, which is not conducive to rapid dosage adjustment (Mark P.Grillo et al. Xenobiotica, 2019; 49(6):718-733). Therefore, developing myosin inhibitors with improved activity and more desirable pharmacokinetic properties holds significant clinical value and implications.

Moreover, anomalies in myocardial sarcomere have been identified as the driving cause behind a variety of heart diseases and symptoms, such as diastolic heart failure with preserved ejection fraction, ischemic heart disease, angina pectoris, and restrictive cardiomyopathy. Myosin ATPase inhibitors, by suppressing myocardial contraction, can also play a potential therapeutic role in alleviating the pathological progression of these diseases.

### CONTENT OF THE PRESENT INVENTION

The present disclosure provides a compound of formula (I) or a pharmaceutically acceptable salt thereof, 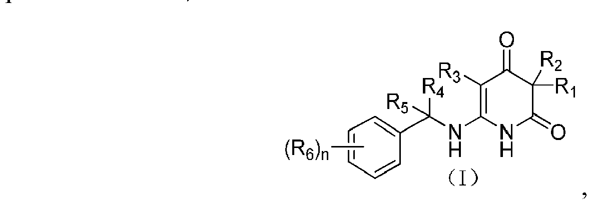 wherein
R₁ and R₂ are each independently selected from H, F, Cl, Br, I, -OH, -NH₂, -CN, C₁₋₄ alkyl, and C₁₋₄ alkoxy, wherein the C₁₋₄ alkyl and C₁₋₄ alkoxy are each independently and optionally substituted by 1, 2, or 3 Rₐ;
alternatively, Ri and R₂ together with the carbon atom to which they are attached form a C₃₋₆ cycloalkyl ring or a 3- to 6-membered heterocycloalkyl ring, wherein the C₃₋₆ cycloalkyl ring and the 3- to 6-membered heterocycloalkyl ring are each independently and optionally substituted by 1, 2, 3, or 4 R_{b};
R₃ is selected from H and F;
R₄ is selected from H, C₁₋₄ alkyl, and C₃₋₄ cycloalkyl, wherein the C₁₋₄ alkyl and C₃₋₄ cycloalkyl are each independently and optionally substituted by 1, 2, or 3 R_{c};
R₅ is selected from H and C₁₋₄ alkyl;
R₆ is selected from H, F, Cl, Br, I, -OH, -NH₂, -CN, C₁₋₄ alkyl, and C₁₋₄ alkoxy, wherein the C₁₋₄ alkyl and C₁₋₄ alkoxy are each independently and optionally substituted by 1, 2, or 3 R_{d};
each Rₐ is independently selected from F, Cl, Br, I, -OH, -NH₂, -CN, C₁₋₄ alkyl, C₁₋₄ alkoxy, -CORₐ₁, -CO₂Rₐ₁, -SO₂Rₐ₁, -SO₂NRₐ₁Rₐ₂, -CONRₐ₁Rₐ₂, wherein the C₁₋₄ alkyl and C₁₋₄ alkoxy are each independently and optionally substituted by 1, 2, or 3 R;
Rₐ₁ and Rₐ₂ are each independently selected from H and C₁₋₄ alkyl;
alternatively, Rₐ₁ and Rₐ₂ together with the nitrogen atom to which they are attached form a 4- to 6-membered heterocycloalkyl ring, wherein the 4- to 6-membered heterocycloalkyl ring is independently and optionally substituted by 1, 2, 3, or 4 Rₑ;
each R_{b} is independently selected from F, Cl, Br, I, -OH, -NH₂, -CN, C₁₋₄ alkyl, C₁₋₄ alkoxy, -COR_{b1}, -CO₂R_{b1}, -SO₂R_{b1}, -SO₂NR_{b1}R_{b2}, and -CONR_{b1}R_{b2}, wherein the C₁₋₄ alkyl and the C₁₋₄ alkoxy are each independently and optionally substituted by 1, 2, or 3 R;
R_{b1} and R_{b2} are each independently selected from H and C₁₋₄ alkyl;
alternatively, R_{b1} and R_{b2} together with the nitrogen atom to which they are attached form a 4- to 6-membered heterocycloalkyl ring, wherein the 4- to 6-membered heterocycloalkyl ring is independently and optionally substituted by 1, 2, 3, or 4 R_{f};
each R_{c} is independently selected from F, Cl, Br, I, -OH, -NH₂, -CN, C₁₋₄ alkyl, and C₁₋₄ alkoxy;
each R_{d} is independently selected from F, Cl, Br, I, -OH, -NH₂, -CN, C₁₋₄ alkyl, and C₁₋₄ alkoxy;
each Rₑ is independently selected from F, Cl, Br, I, -OH, -NH₂, -CN, C₁₋₄ alkyl, and C₁₋₄ alkoxy;
each R_{f} is independently selected from F, Cl, Br, I, -OH, -NH₂, -CN, C₁₋₄ alkyl, and C₁₋₄ alkoxy;
each R is independently selected from F, Cl, Br, I, -OH, -NH₂, and -CN;
n is selected from 1, 2, 3, or 4;
the 3- to 6-membered heterocycloalkyl ring and the 4- to 6-membered heterocycloalkyl ring each independently include 1, 2, 3, or 4 atoms or atomic groups each independently selected from N, O, S, and NH.

The present disclosure provides a compound of formula (I) or a pharmaceutically acceptable salt thereof, 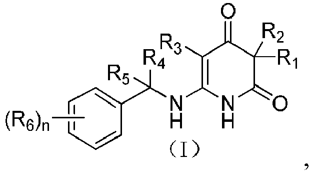 wherein
R₁ and R₂ are each independently selected from H, F, Cl, Br, I, -OH, -NH₂, -CN, and C₁₋₄ alkyl;
alternatively, R₁ and R₂ together with the carbon atom to which they are attached form a C₄₋₆ cycloalkyl ring or a 5- to 6-membered heterocycloalkyl ring, wherein the C₄₋₆ cycloalkyl ring and the 5- to 6-membered heterocycloalkyl ring are each independently and optionally substituted by 1, 2, 3, or 4 R_{b};
R₃ is selected from H and F;
R₄ is selected from H and C₁₋₄ alkyl;
R₅ is selected from H;
R₆ is selected from H, F, Cl, Br, I, and C₁₋₄ alkyl;
each R_{b} is independently selected from F, Cl, Br, I, -OH, -NH₂, -CN, C₁₋₄ alkoxy, - COR_{b1}, and -CO₂R_{b1};
R_{b1} is selected from H and C₁₋₄ alkyl;
n is selected from 1 or 2;
the 5- to 6-membered heterocycloalkyl ring include 1, 2, 3, or 4 atoms or atomic groups each independently selected from N, O, S, and NH.

In some embodiments of the present disclosure, the Rₐ₁ and Rₐ₂ are each independently selected from H, and other variables are as defined in the present disclosure.

In some embodiments of the present disclosure, the Rₐ, R_{c}, R_{d}, Rₑ, and R_{f} are each independently selected from F and Cl, and other variables are as defined in the present disclosure.

In some embodiments of the present disclosure, the R₁ and R₂ are each independently selected from -CH₃ and -CH₂CH₃, wherein the -CH₃ and -CH₂CH₃ are each independently and optionally substituted by 1, 2, or 3 Rₐ, and the Rₐ and other variables are as defined in the present disclosure.

In some embodiments of the present disclosure, the R₁ and R₂ are each independently selected from -CH₃ and -CH₂CH₃, and other variables are as defined in the present disclosure.

In some embodiments of the present disclosure, the R_{b1} and R_{b2} are each independently selected from -CH₃ and -CH₂CH₃, and other variables are as defined in the present disclosure.

In some embodiments of the present disclosure, each R_{b} is independently selected from F, Cl, Br, -OCH₃, -COCH₃, -CO₂CH₃, and -CO₂CH₂CH₃, and other variables are as defined in the present disclosure.

In some embodiments of the present disclosure, each R_{b} is independently selected from F, Cl, Br, -OCH₃, -COCH₃, and -CO₂CH₂CH₃, and other variables are as defined in the present disclosure.

In some embodiments of the present disclosure, the R₁ and R₂ together with the carbon atom to which they are attached form a C₅₋₆ cycloalkyl ring or a 6-membered heterocycloalkyl ring, wherein the C₅₋₆ cycloalkyl ring and the 6-membered heterocycloalkyl ring are each independently and optionally substituted by 1, 2, 3, or 4 R_{b}, and R_{b} and other variables are as defined in the present disclosure.

In some embodiments of the present disclosure, the R₁ and R₂ together with the carbon atom to which they are attached form 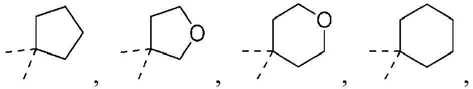 or 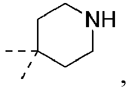 wherein the 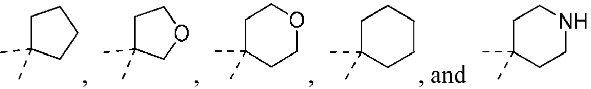 are each independently and optionally substituted by 1, 2, 3, or 4 R_{b}, and R_{b} and other variables are as defined in the present disclosure.

In some embodiments of the present disclosure, the R₁ and R₂ together with the carbon atom to which they are attached form 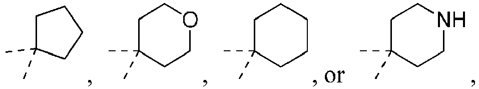 wherein the 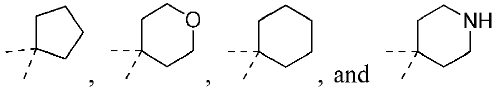 are each independently and optionally substituted by 1, 2, 3, or 4 R_{b}, and R_{b} and other variables are as defined in the present disclosure.

In some embodiments of the present disclosure, the R₁ and R₂ together with the carbon atom to which they are attached form 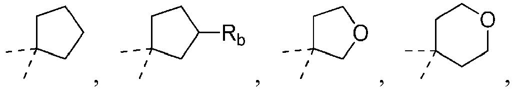 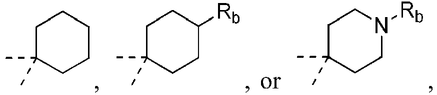 and R_{b} and other variables are as defined in the present disclosure.

In some embodiments of the present disclosure, the R₁ and R₂ together with the carbon atom to which they are attached form 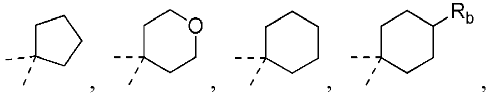 or 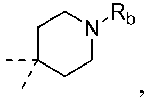 and R_{b} and other variables are as defined in the present disclosure.

In some embodiments of the present disclosure, the R₁ and R₂ together with the carbon atom to which they are attached form 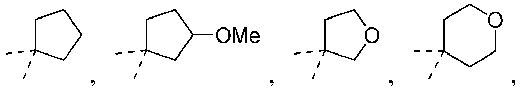 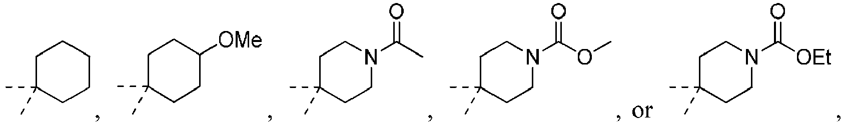 and other variables are as defined in the present disclosure.

In some embodiments of the present disclosure, the R₁ and R₂ together with the carbon atom to which they are attached form 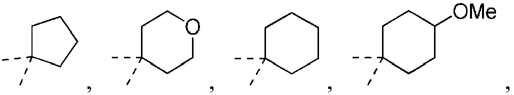 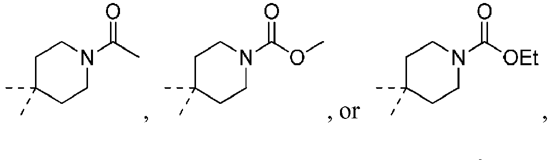 and other variables are as defined in the present disclosure.

In some embodiments of the present disclosure, the structural moiety 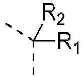 is selected from 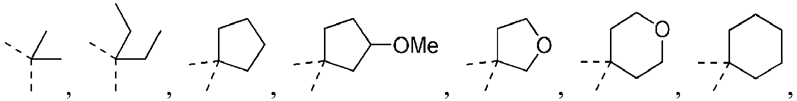 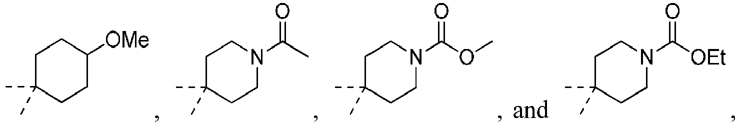 and other variables are as defined in the present disclosure.

In some embodiments of the present disclosure, the structural moiety 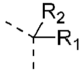 is selected from 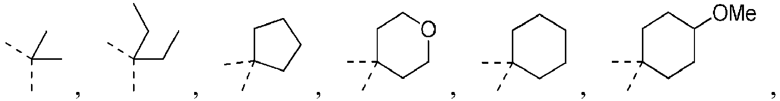 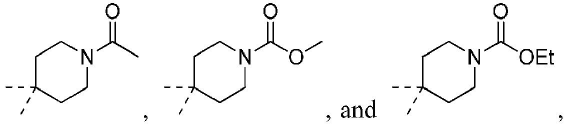 and other variables are as defined in the present disclosure.

In some embodiments of the present disclosure, the structural moiety 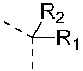 is selected from 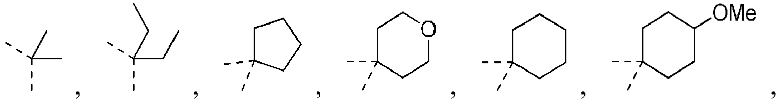 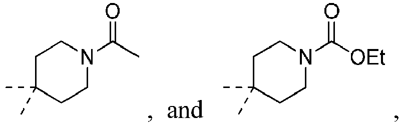 and other variables are as defined in the present disclosure.

In some embodiments of the present disclosure, the R₃ is selected from H, and other variables are as defined in the present disclosure.

In some embodiments of the present disclosure, the R₄ is selected from C₁₋₄ alkyl, and other variables are as defined in the present disclosure.

In some embodiments of the present disclosure, the R₄ is selected from -CH₃ and - CH₂CH₃, wherein the -CH₃ and -CH₂CH₃ are each independently and optionally substituted by 1, 2, or 3 R_{d}, and the R_{d} and other variables are as defined in the present disclosure.

In some embodiments of the present disclosure, the R₄ is selected from -CH₃ and - CH₂CH₃, and other variables are as defined in the present disclosure.

In some embodiments of the present disclosure, the R₄ is selected from -CH₃, and other variables are as defined in the present disclosure.

In some embodiments of the present disclosure, the R₅ is selected from H, and other variables are as defined in the present disclosure.

In some embodiments of the present disclosure, each R₆ is independently selected from H, F, Cl, and -CH₃, wherein the -CH₃ is optionally substituted by 1, 2, or 3 R_{d}, and R_{d} and other variables are as defined in the present disclosure.

In some embodiments of the present disclosure, each R₆ is independently selected from H, F, Cl, and -CH₃, and other variables are as defined in the present disclosure.

In some embodiments of the present disclosure, each R₆ is independently selected from H, F, and -CH₃, and other variables are as defined in the present disclosure.

In some embodiments of the present disclosure, the compound has a structure of formula (I-1): 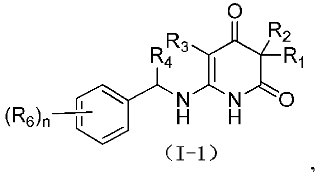 wherein n, R₁, R₂, R₃, R₄, and R₆ are as defined in the present disclosure.

In some embodiments of the present disclosure, the compound has a structure of formula (I-1-1): 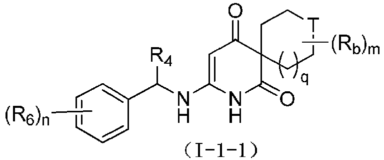 wherein
n is selected from 1 and 2;
m is selected from 0, 1, and 2;
q is selected from 0 and 1;
T is selected from CH₂, O, and NH, and when T is selected from CH₂ and NH, T can be optionally substituted by R_{b};
R_{b}, R₄, and R₆ are as defined in the present disclosure.

In some embodiments of the present disclosure, the compound has a structure of formula (I-1A) or formula(I-1B): 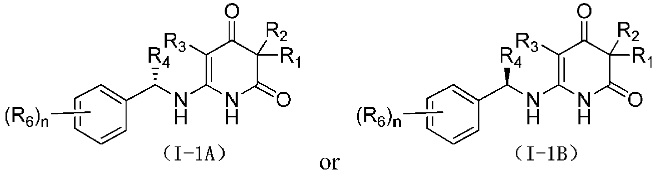 wherein n, R₁, R₂, R₃, R₄, and R₆ are as defined in the present disclosure, and R₄ is not H.

In some embodiments of the present disclosure, the compound has a structure of formula (I-1-1A) or formula(I-1-1B): 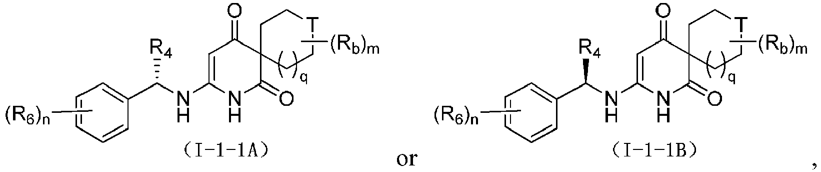 wherein
n is selected from 1 and 2;
m is selected from 0, 1, and 2;
q is selected from 0 and 1;
R₄ is selected from C₁₋₄ alkyl;
T is selected from CH₂, O, and NH, and when T is selected from CH₂ and NH, T can be optionally substituted by R_{b};
R_{b}, R₄, and R₆ are as defined in the present disclosure.

There are still some embodiments of the present disclosure which are obtained by any combination of the above variables.

The present disclosure also provides a compound of the following formula or a pharmaceutically acceptable salt thereof,

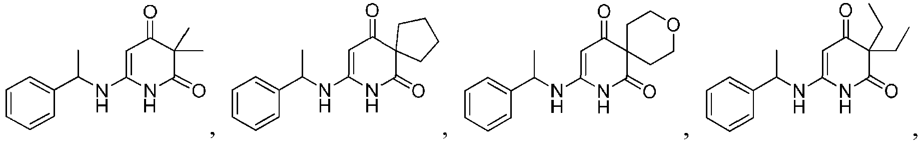

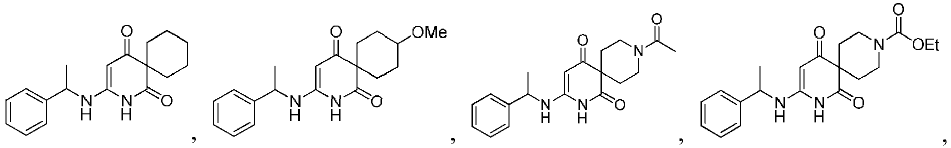

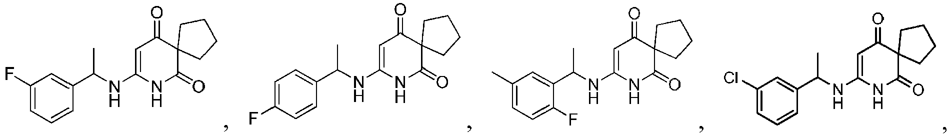

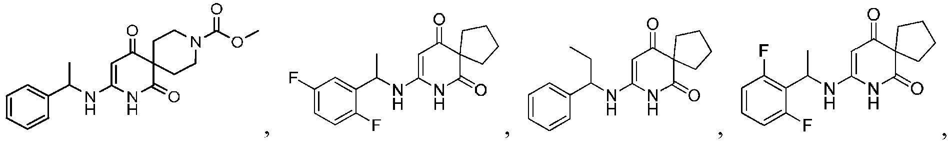

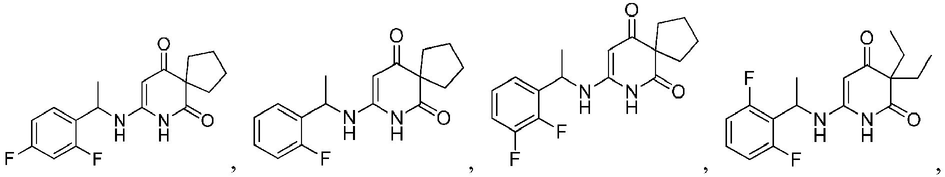

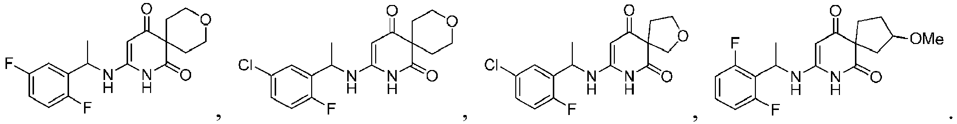

The present disclosure also provides a compound of the following formula or a pharmaceutically acceptable salt thereof,

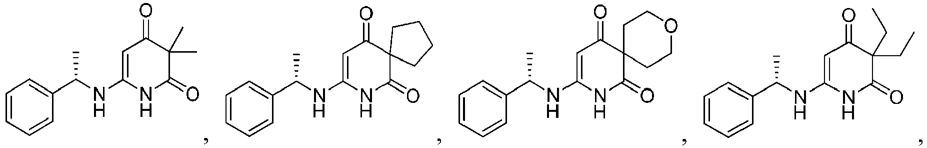

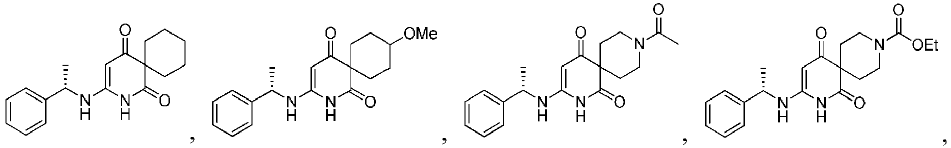

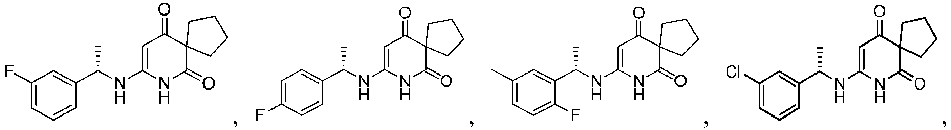

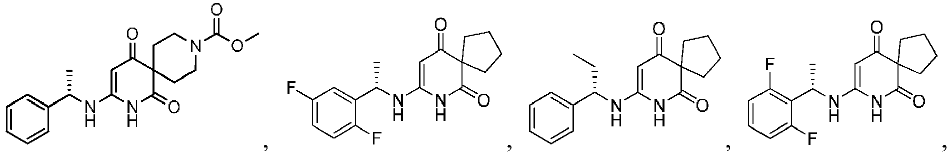

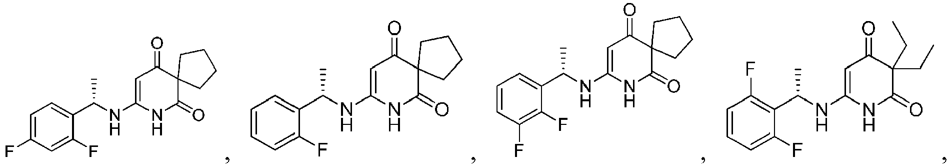

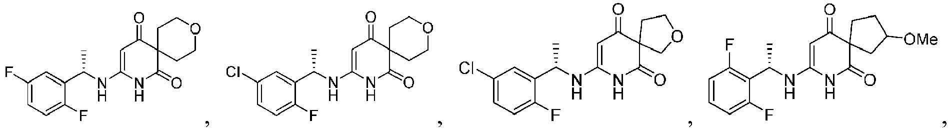

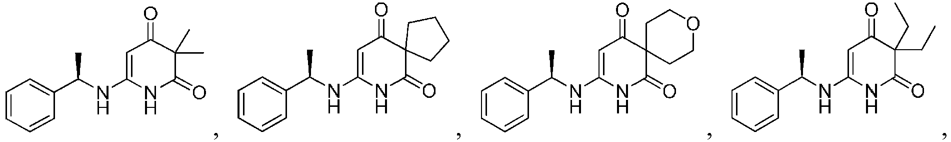

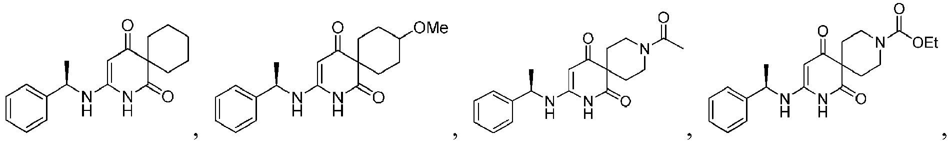

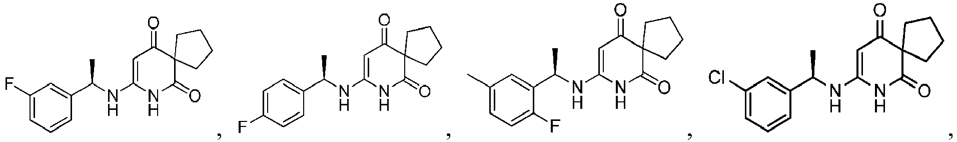

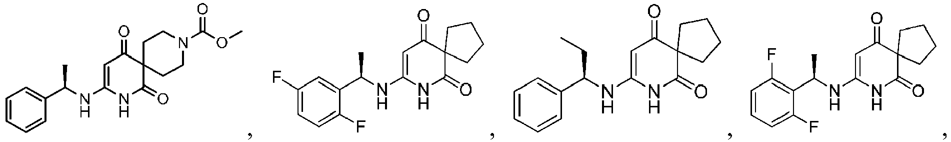

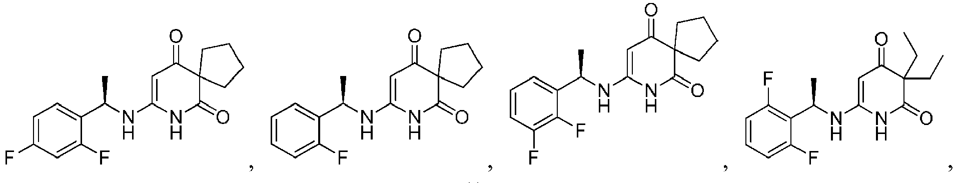

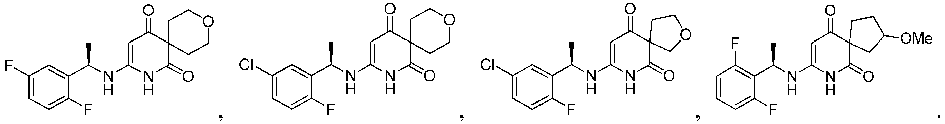

The present disclosure also provides a pharmaceutical composition comprising a therapeutically effective amount of the compound or the pharmaceutically acceptable salt thereof and a pharmaceutically acceptable carrier.

The present disclosure also provides a use of the compound, the pharmaceutically acceptable salt thereof, or the pharmaceutical composition in the manufacture of a cardiac myosin inhibitor medicament.

The present disclosure also provides a use of the compound, the pharmaceutically acceptable salt thereof, or the pharmaceutical composition in the manufacture of a medicament for treating heart failure and hypertrophic cardiomyopathy.

The present disclosure also provides a method for treating cardiac myosin inhibitor-related diseases in a subject in need thereof; the method includes administering to the subject an effective amount of the compound or the pharmaceutically acceptable salt thereof defined in any of the technical solutions, or the pharmaceutical composition.

The present disclosure also provides a method for treating heart failure and hypertrophic cardiomyopathy in a subject in need thereof; the method includes administering to the subject an effective amount of the compound or the pharmaceutically acceptable salt thereof defined in any of the technical solutions, or the pharmaceutical composition.

### Technical effect

The compounds of the present disclosure have a good inhibitory effect on cardiac myosin ATPase and demonstrate excellent pharmacokinetic properties.

### Definition and description

Unless otherwise specified, the following terms and phrases used herein have the following meanings. A specific term or phrase should not be considered indefinite or unclear in the absence of a particular definition, but should be understood according to the common meaning. When a trade name appears herein, it is intended to refer to its corresponding commercial product or active ingredient thereof.

The term "pharmaceutically acceptable" is used herein in terms of those compounds, materials, compositions, and/or dosage forms, which are suitable for use in contact with human and animal tissues within the scope of reliable medical judgment, without excessive toxicity, irritation, anaphylactic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting the compound with a sufficient amount of a base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, an acid addition salt can be obtained by bringing the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of an amino acid (such as arginine), and a salt of an organic acid such as glucuronic acid. Certain specific compounds of the present disclosure contain both basic and acidic functional groups, and thus can be converted to any base or acid addition salt.

The pharmaceutically acceptable salt of the present disclosure can be prepared from the parent compound that contains an acidic or basic moiety by a conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)- and (+)-enantiomers, (R)- and (*S*)-enantiomers, diastereoisomers, (D)-isomers, (Z)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid bond ( 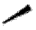 ) and a wedged dashed bond ( 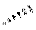 ), and the relative configuration of a stereogenic center is represented by a straight solid bond ( 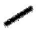 ) and a straight dashed bond ( 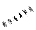 ), a wave line ( 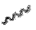 ) is used to represent a wedged dashed bond ( 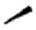 ) or a wedged dashed bond ( 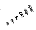 ), or the wave line ( 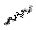 ) is used to represent a straight solid bond ( 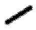 ) and a straight dashed bond ( 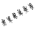 ).

The compounds of the present disclosure may be specific. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imineenamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (R)- and (*S*)-isomer, *or D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom(s) that constitute the compound. For example, the compound can be radiolabeled with a radioactive isotope, such as tritium (³H), iodine-125 (¹²⁵I), or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequently described event or circumstance may, but does not necessarily, occur, and the description includes instances where the event or circumstance occurs and instances where it does not.

The term "substituted" means one or more than one hydrogen atom(s) on a specific atom are substituted by the substituent, including deuterium and hydrogen variables, as long as the valence of the specific atom is normal and the substituted compound is stable. When the substituent is oxygen (i.e., =O), it means two hydrogen atoms are substituted. Positions on an aromatic ring cannot be substituted by a ketone.

The term "optionally substituted" means an atom can be substituted by a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent is absent, for example, when X is vacant in A-X, the structure of A-X is actually A. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 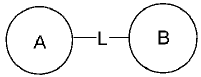 is -M-W-, then -M-W- can link ring A and ring B to form 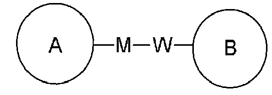 in the direction same as left-to-right reading order, and form 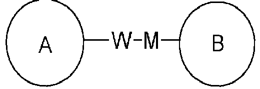 in the direction contrary to left-to-right reading order. A combination of the linking groups, substituents, and/or variables thereof is allowed only when such combination can result in a stable compound.

Unless otherwise specified, when a group has one or more than one linkable site, any one or more than one site of the group can be linked to other groups through chemical bonds. When the linking site of the chemical bond is not positioned, and there is an H atom at the linkable site, then the number of H atoms at the site will decrease correspondingly with the number of chemical bonds linking thereto so as to meet the corresponding valence. The chemical bond between the site and other groups can be represented by a straight solid bond ( 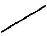 ), a straight dashed bond ( 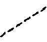 ), or a wavy line ( 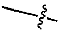 ). For example, the straight solid bond in -OCH₃ means that it is linked to other groups through the oxygen atom in the group; the straight dashed bond in 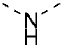 means that it is linked to other groups through the two ends of the nitrogen atom in the group; the wave lines in 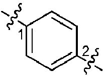 means that the phenyl group is linked to other groups through carbon atoms at position 1 and position 2. 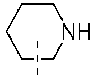 means that it can be linked to other groups through any linkable sites on the piperidinyl by one chemical bond, including at least four types of linkage, including 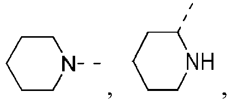 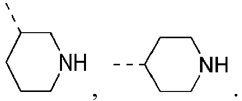 Even though the H atom is drawn on the -N-, 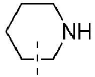 still includes the linkage of 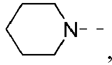 merely when one chemical bond was connected, the H of this site will be reduced by one to the corresponding monovalent piperidinyl.

When the chemical bond of a substituent intersects with the chemical bonds connecting two atoms on a ring, it indicates that the substituent can bond with any atom on the ring. When the atom to which a substituent is connected is not specified, the substituent can bond with any atom. If the atom connected to the substituent is in a bicyclic or tricyclic system, it indicates that the substituent can bond with any atom in any ring of that system. A combination of the substituents and/or variables thereof is allowed only when such combination can result in a stable compound. For example, structural moiety 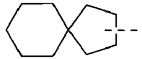 or 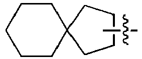 indicates that it can be substituted at any position on cyclohexyl or cyclopentyl.

Unless otherwise specified, the number of atoms in a ring is usually defined as the number of ring members, for example, "5- to 7-membered ring" refers to a "ring" in which 5 to 7 atoms are arranged around.

Unless otherwise specified, the term "C₁₋₃ alkyl" refers to a linear or branched saturated hydrocarbon group consisting of 1 to 3 carbon atoms. The C₁₋₃ alkyl includes C₁₋₂ and C₂₋₃ alkyl, etc.; it can be monovalent (such as methyl), divalent (such as methylene), or multivalent (such as methine). Examples of C₁₋₃ alkyl include, but are not limited to methyl (Me), ethyl (Et), propyl (including n-propyl and isopropyl), etc.

Unless otherwise specified, the term "C₁₋₄ alkyl" refers to a linear or branched saturated hydrocarbon group consisting of 1 to 4 carbon atoms. The C₁₋₄ alkyl includes C₁₋₂, C₁₋₃, and C₂₋₃ alkyl, etc.; it can be monovalent (such as methyl), divalent (such as methylene), or multivalent (such as methine). Examples of C₁₋₄ alkyl include, but are not limited to, methyl (Me), ethyl (Et), propyl (including n-propyl and isopropyl), butyl (including n-butyl, isobutyl, s-butyl, and t-butyl), etc.

Unless otherwise specified, the term "C₁₋₄ alkoxy" refers to an alkyl group containing 1 to 4 carbon atoms that are connected to the rest of the molecule through an oxygen atom. The C₁₋₄ alkoxy includes C₁₋₃, C₁₋₂, C₂₋₄, C₄, and C₃ alkoxy, etc. Examples of C₁₋₄ alkoxy include, but are not limited to, methoxy, ethoxy, propoxy (including n-propoxy and isopropoxy), and butoxy (including n-butoxy, isobutoxy, s-butoxy, and t-butoxy).

The term "heteroalkyl" itself or in combination with other terms, refers to a stable linear or branched alkyl atomic group or its combination, consisting of a certain number of carbon atoms and at least one heteroatom or heteroatom group. In some embodiments, the heteroatom is selected from B, O, N, and S, wherein nitrogen and sulfur atoms are optionally oxidized, and nitrogen heteroatoms are optionally quaternized. In other embodiments, the heteroatom group is selected from -C(=O)O-, -C(=O)-, -C(=S)-, -S(=O), -S(=O)₂-, - C(=O)N(H)-, -N(H)-, -C(=NH)-, -S(=O)₂N(H)-, and -S(=O)N(H)-. In some embodiments, the heteroalkyl is C₁₋₆ heteroalkyl; in other embodiments, the heteroalkyl is C₁₋₃ heteroalkyl. Heteroatoms or heteroatom groups can be located at any internal position within the heteroalkyl, including the position where the alkyl is connected to the rest of the molecule. However, the terms "alkoxy," "alkylamino," and "alkylthio" (or "thioalkoxy") are common expressions that specifically refer to alkyl groups connected to the rest of the molecule through an oxygen atom, an amino group, or a sulfur atom, respectively. Examples of the heteroalkyl include, but are not limited to, -OCH₃, -OCH₂CH₃, -OCH₂CH₂CH₃, -OCH₂(CH₃)₂, -CH₂-CH₂-O-CH₃, - NHCH₃, -N(CH₃)₂, -NHCH₂CH₃, -N(CH₃)(CH₂CH₃), -CH₂-CH₂-NH-CH₃, -CH₂-CH₂-N(CH₃)-CH₃, -SCH₃, -SCH₂CH₃, -SCH₂CH₂CH₃, -SCH₂(CH₃)₂, -CH₂-S-CH₂-CH₃, -CH₂-CH₂, -S(=O)-CH₃, -CH₂-CH₂-S(=O)₂-CH₃. Up to two heteroatoms can be connected in a row, for example, -CH₂-NH-OCH₃.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, Cs, C₉, C₁₀, C₁₁, and C₁₂, and any range from n to n+m is also included, for example, C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂, etc.; similarly, n-membered to n+m-membered means that the number of atoms on the ring is from n to n+m, for example, 3- to 12-membered ring includes 3-membered ring, 4-membered ring, 5-membered ring, 6-membered ring, 7-membered ring, 8-membered ring, 9-membered ring, 10-membered ring, 11-membered ring, and 12-membered ring, and any range from n to n+m is also included, for example, 3- to 12-membered ring includes 3- to 6-membered ring, 3- to 9-membered ring, 5- to 6-membered ring, 5- to 7-membered ring, 6- to 7-membered ring, 6- to 8-membered ring, and 6- to 10-membered ring, etc.

Unless otherwise specified, "C₃₋₆ cycloalkyl" refers to a saturated cyclic hydrocarbon group consisting of 3 to 6 carbon atoms, which can be monocyclic and bicyclic, and the C₃₋₆ cycloalkyl includes C₃₋₅, C₄₋₅, and C₅₋₆ cycloalkyl, etc.; it can be monovalent, divalent, or multivalent. Examples of C₃₋₆ cycloalkyl include, but are not limited to, cyclopropyl, cyclobutyl, cyclopentyl, cyclohexyl, etc.

Unless otherwise specified, the term "C₃₋₄ cycloalkyl" refers to a saturated cyclic hydrocarbon group consisting of 3 to 4 carbon atoms, which is monocyclic; it can be monovalent, divalent, or multivalent. Examples of C₃₋₅ cycloalkyl include, but are not limited to, cyclopropyl, cyclobutyl, cyclopentyl, etc.

Unless otherwise specified, "C₄₋₆ cycloalkyl" refers to a saturated cyclic hydrocarbon group consisting of 4 to 6 carbon atoms, which can be monocyclic and bicyclic, and the C₄₋₆ cycloalkyl includes C₄₋₅ cycloalkyl, C₅₋₆ cycloalkyl, etc.; it can be monovalent, divalent, or multivalent. Examples of C₄₋₆ cycloalkyl include, but are not limited to, cyclobutyl, cyclopentyl, cyclohexyl, etc.

Unless otherwise specified, "C₅₋₆ cycloalkyl" refers to a saturated hydrocarbon group consisting of 5 to 6 carbon atoms, which can be monocyclic and bicyclic, and the C₃₋₆ cycloalkyl includes 5-membered cycloalkyl, 6-membered cycloalkyl, etc.; it can be monovalent, divalent, or multivalent. Examples of C₅₋₆ cycloalkyl include, but are not limited to, cyclopentyl, cyclohexyl, etc.

Unless otherwise specified, the term "3- to 6-membered heterocycloalkyl" by itself or in combination with other terms refers to a saturated cyclic group consisting of 3 to 6 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)ₚ, p is 1 or 2). It includes monocyclic and bicyclic systems, wherein the bicyclic system includes a spiro ring, a fused ring, and a bridged ring. In addition, with regard to the "3- to 6-membered heterocycloalkyl", a heteroatom may occupy the connection position of the heterocycloalkyl with the rest of the molecule. The 3- to 6-membered heterocycloalkyl includes 4- to 6-membered, 5- to 6-membered, 4-membered, 5-membered, and 6-membered heterocycloalkyl, etc. Examples of 3- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, or hexahydropyridazinyl, etc.

Unless otherwise specified, the term "4- to 6-membered heterocycloalkyl" by itself or in combination with other terms refers to a saturated cyclic group consisting of 4 to 6 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)ₚ, p is 1 or 2). It includes monocyclic and bicyclic systems, wherein the bicyclic system includes a spiro ring, a fused ring, and a bridged ring. In addition, with regard to the "4- to 6-membered heterocycloalkyl", a heteroatom may occupy the connection position of the heterocycloalkyl with the rest of the molecule. The 4- to 6-membered heterocycloalkyl includes 5- to 6-membered, 4-membered, 5-membered, and 6-membered heterocycloalkyl, etc. Examples of 4- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, or hexahydropyridazinyl, etc.

Unless otherwise specified, the term "5- to 6-membered heterocycloalkyl" by itself or in combination with other terms refers to a saturated cyclic group consisting of 5 to 6 ring atoms, wherein 1, 2, 3, or 4 ring atoms are heteroatoms independently selected from O, S, and N, and the rest are carbon atoms, wherein nitrogen atoms are optionally quaternized, and carbon, nitrogen, and sulfur heteroatoms can be optionally oxidized (i.e. C(=O), NO, and S(O)ₚ, and p is 1 or 2). It includes monocyclic and bicyclic systems, wherein the bicyclic system includes a spiro ring, a fused ring, and a bridged ring. In addition, with regard to the "5- to 6-membered heterocycloalkyl", a heteroatom may occupy the connection position of the heterocycloalkyl with the rest of the molecule. The 5- to 6-membered heterocycloalkyl includes 5-membered and 6-membered heterocycloalkyl. Examples of 5- to 6-membered heterocycloalkyl include, but are not limited to, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, or hexahydropyridazinyl, etc.

Unless otherwise specified, the term "6-membered heterocycloalkyl" by itself or in combination with other terms refers to a saturated cyclic group consisting of 6 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (i.e. C(=O), NO and S(O)ₚ, p is 1 or 2). It includes monocyclic and bicyclic systems, wherein the bicyclic system includes a spiro ring, a fused ring, and a bridged ring. In addition, in terms of the "6-membered heterocycloalkyl", the heteroatom may occupy the connection position of the heterocycloalkyl with the rest of the molecule. Examples of 6-membered heterocycloalkyl include, but are not limited to, tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, or hexahydropyridazinyl, etc.

The term "leaving group" refers to a functional group or atom which can be substituted by another functional group or atom through a substitution reaction (such as nucleophilic substitution reaction). For example, representative leaving groups include triflate; chlorine, bromine, and iodine; sulfonate group, such as mesylate, tosylate, p-bromobenzenesulfonate, p-toluenesulfonates, etc.; acyloxy, such as acetoxy, trifluoroacetoxy, etc.

The term "protecting group" includes, but is not limited to "amino protecting group", "hydroxy protecting group", or "thio protecting group". The term "amino protecting group" refers to a protecting group suitable for blocking the side reaction on the nitrogen of an amino. Representative amino protecting groups include, but are not limited to: formyl; acyl, such as alkanoyl (e.g., acetyl, trichloroacetyl or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS). The term "hydroxyl protecting group" refers to a protecting group suitable for preventing the side reactions of hydroxyl. Representative hydroxyl protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyl dimethyl silyl (TBS).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art. Preferred embodiments include but are not limited to the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: ϕ/ω scan, and after collecting the relevant data, the crystal structure is further analyzed by direct method (Shelxs97), so that the absolute configuration can be confirmed.

The solvent used in the present disclosure is commercially available.

The present disclosure uses the following abbreviations: TEA stands for triethylamine; DIEA stands for N, N-diisopropylethylamine; PE stands for petroleum ether; EtOAc stands for ethyl acetate; EA stands for ethyl acetate; THF stands for tetrahydrofuran; MeOH stands for methanol; MTBE stands for methyl tert-butyl ether; DCM stands for dichloromethane; EtOH stands for ethanol; iPrOH stands for isopropanol; Boc₂O stands for di-tert-butyl dicarbonate; L-selectride stands for lithium triisobutylhydroborate; TCFH stands for N,N,N,N-tetramethylchloroformamidinium hexafluorophosphate; FA stands for formic acid; TFA stands for trifluoroacetic acid; ACN stands for acetonitrile; TLC stands for thin-layer chromatography; HPLC stands for high performance liquid chromatography; LCMS stands for liquid chromatography-mass spectrometry. DMSO stands for dimethyl sulfoxide; DMF stands for *N,N*-dimethylformamide; LDA stands for lithium diisopropylamide; DMAC stands for *N,N-*dimethylacetamide; PEG-400 stands for polyethylene glycol 400; EGTA stands for ethylene glycol bis(2-aminoethyl ether) tetraacetic acid; DMSO-*d*₆ stands for deuterated dimethyl sulfoxide; CDCl₃ stands for deuterated chloroform.

The compounds of the present disclosure are named according to the conventional naming principles in the art or by ChemDraw^{®} software, and the commercially available compounds use the supplier catalog names.

### DETAILED DESCRIPTION OF THE PREFERRED EMBODIMENT

The present disclosure is described in detail by the examples below, but it does not mean that there are any adverse restrictions on the present disclosure. The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art. Preferred embodiments include but are not limited to the examples of the present disclosure. For one skilled in the art, it is obvious to make various modifications and improvements to the embodiments of the present disclosure without departing from the spirit and scope of the present disclosure.

### Example 1

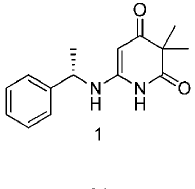

### Synthetic route:

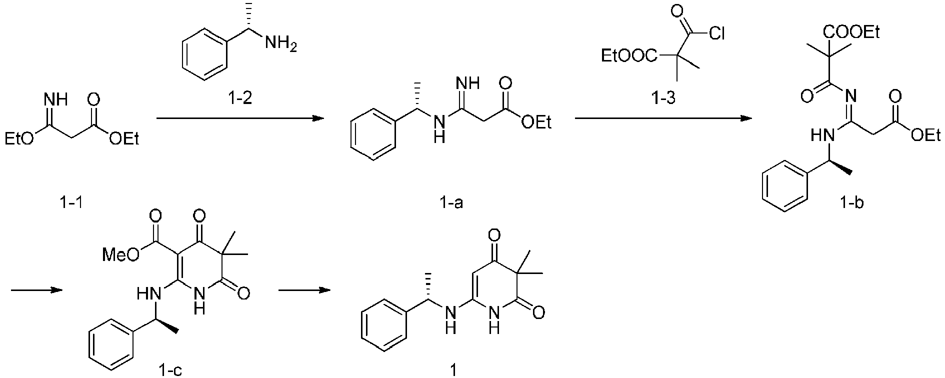

Step A: At 20°C, to a solution of compound 1-2 (929.08 mg, 7.67 mmol, 975.93 µL, 1 eq) and compound 1-1 (1.5 g, 7.67 mmol, 1 eq, HCL) in EtOH (20 mL) was added DIEA (1.98 g, 15.33 mmol, 2.67 mL, 2 eq). The reaction mixture was stirred at 20°C for 16 hours, then concentrated to obtain compound 1-a.

Step B: Under nitrogen atmosphere at 0°C, to a solution of compound 1-a (1.6 g, 6.83 mmol, 1 eq) in THF (30 mL) was added DIEA (1.77 g, 13.66 mmol, 2.38 mL, 2 eq) and compound 1-3 (1.34 g, 7.51 mmol, 1.1 eq). The reaction mixture was stirred at 20°C for 1 hour, then concentrated. The residue was purified by silica gel column chromatography (PE: EtOAc = 10:1 to 3:1) to obtain compound 1-b. LCMS(ESI) m/z: 377.3(M+1).

Step C: Under nitrogen atmosphere, to a solution of compound 1-b (1.5 g, 3.98 mmol, 1 eq) in MeOH (30 mL) was added sodium tert-butoxide (1.53 g, 15.94 mmol, 4 eq). The reaction mixture was stirred at 20°C for 4 hours, then added with dilute hydrochloric acid (1 mol/L, 50 mL), and was extracted with EA (50 mL). The organic phase was washed with saturated brine (50 mL), dried over anhydrous sodium sulfate, filtered, and concentrated. The residue was purified by silica gel column chromatography (PE: EtOAc = 4:1 to 2:1) to obtain compound 1-c.

Step D: Under nitrogen atmosphere, to a solution of compound 1-c (0.4 g, 1.26 mmol, 1 eq) in DMSO (4 mL) was added lithium chloride (107.20 mg, 2.53 mmol, 51.79 µL, 2 eq). The reaction mixture was stirred at 125°C for 20 hours, then filtered. The filtrate was purified by preparative HPLC [mobile phase: water (0.1% TFA)-ACN; gradient: 21% to 51% ACN] to obtain compound 1. ¹H NMR (CDCl₃, 400 MHz): 7.38 - 7.24 (m, 5H), 5.17 (br s, 1H), 4.63 (br d, J=6.4 Hz, 1H), 1.57 (d, J=6.7 Hz, 3H), 1.42 (s, 3H), 1.38 (s, 3H); LCMS(ESI) m/z: 259.4(M+1).

### Example 2

### Synthetic route:

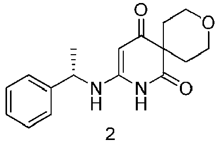

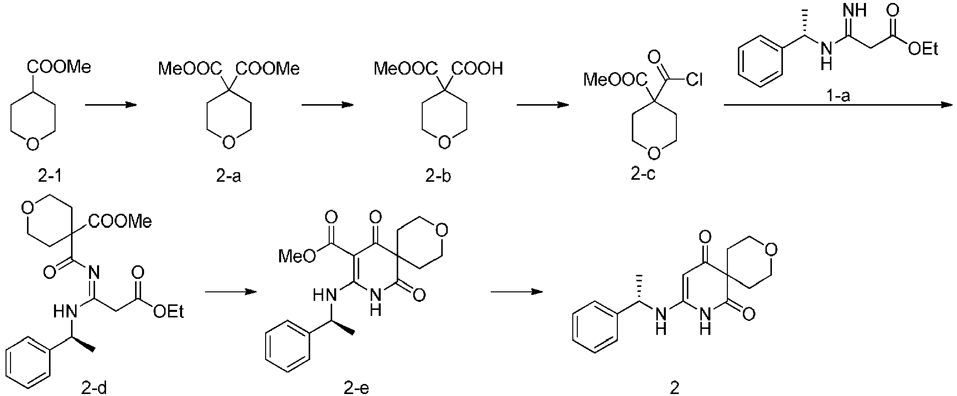

Step A: Under nitrogen atmosphere at -78°C, to a solution of compound 2-1 (5 g, 34.68 mmol, 4.63 mL, 1 eq) in THF (80 mL) was dropwise added LDA (2 M, 19.07 mL, 1.1 eq). The reaction mixture was stirred at -78°C for 30 minutes, and then methyl chloroformate (3.44 g, 36.42 mmol, 2.82 mL, 1.05 eq) was added thereto. The reaction mixture was slowly warmed to 20°C, stirred for 16 hours, quenched with water (200 mL), and extracted with EA (200 mL). The organic phase was washed with saturated brine (200 mL), dried over anhydrous sodium sulfate, filtered, and concentrated. The residue was purified by silica gel column chromatography (PE: EtOAc = 10:1 to 3:1) to obtain compound 2-a.

Step B: At 0°C, to a solution of compound 2-a (2.5 g, 12.36 mmol, 1 eq) in MeOH (20 mL) and water (20 mL) was added sodium hydroxide (494.51 mg, 12.36 mmol, 1 eq). The reaction mixture was stirred at 20°C for 16 hours, then added with water (50 mL), and extracted with EA (50 mL). After separating the phases, the aqueous phase was added with 1 M dilute hydrochloric acid to adjust the pH to around 5, and then extracted with EA (50 mL). The organic phase was washed with saturated brine (50 mL), dried over anhydrous sodium sulfate, filtered, and concentrated to obtain compound 2-b.

Step C: Under nitrogen atmosphere at 0°C, to a solution of compound 2-b (1.7 g, 9.03 mmol, 1 eq) in DCM (30 mL) was added TEA (4.57 g, 45.17 mmol, 6.29 mL, 5 eq) and DMF (33.02 mg, 451.70 µmol, 34.75 µL, 0.05 eq), then oxalyl chloride (1.72 g, 13.55 mmol, 1.19 mL, 1.5 eq) was added thereto. The reaction mixture was stirred at 20°C for 1 hour, and concentrated to obtain compound 2-c.

Step D: Under nitrogen atmosphere at 0°C, to a solution of compound 2-c (2.0 g, 9.68 mmol, 1 eq) in DCM (30 mL) was added DIEA (2.50 g, 19.36 mmol, 3.37 mL, 2 eq) and compound 1-a (2.27 g, 9.68 mmol, 1 eq). The reaction mixture was stirred at 20°C for 1 hour, then concentrated, diluted with EA (30 mL), washed with 1 N dilute hydrochloric acid (30 mL), and then washed with saturated brine (30 mL). The mixture was dried over anhydrous sodium sulfate, filtered, and concentrated. The residue was purified by silica gel column chromatography (PE: EtOAc = 10:1 to 3:1) to obtain compound 2-d.

Step E: Under nitrogen atmosphere, to a solution of compound 2-d (0.35 g, 865.36 µmol, 1 eq) in MeOH (5 mL) was added sodium methoxide (2 M, 7 mL, 16.18 eq). The reaction mixture was stirred at 50°C for 2 hours, and concentrated. The residue was diluted with EA (20 mL), washed with saturated brine (20 mL), dried over anhydrous sodium sulfate, filtered, and concentrated. The residue was purified by silica gel chromatography (PE: EtOAc = 10:1 to 1:1) to obtain compound 2-e.

Step F: To a solution of compound 2-e (70 mg, 195.32 µmol, 1 eq) in 1,4-dioxane (7 mL) was added hydrochloric acid (4 M, 7.00 mL, 143.35 eq). The reaction mixture was stirred at 50°C for 16 hours, and concentrated. The residue was adjusted to neutrality by the addition of 2N sodium hydroxide aqueous solution, then extracted with EA (50 mL). The organic phase was washed with saturated brine (30 mL), dried over anhydrous sodium sulfate, filtered, and concentrated. The residue was purified by preparative HPLC ([water (0.225% FA)-ACN]; gradient: 17% to 47% ACN) to obtain compound 2. ¹H NMR (DMSO-*d₆*, 400 MHz): δ ppm 9.62 (br s, 1H), 7.47 - 7.20 (m, 5H), 7.05 (br s, 1H), 4.60 (br t, J=6.7 Hz, 1H), 4.41 (s, 1H), 3.85 - 3.66 (m, 4H), 1.94 - 1.75 (m, 2H), 1.68 - 1.51 (m, 2H), 1.43 (d, J=6.8 Hz, 3H); LCMS(ESI) m/z: 301.4(M+1).

### Example 3

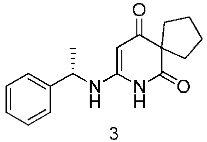

### Synthetic route:

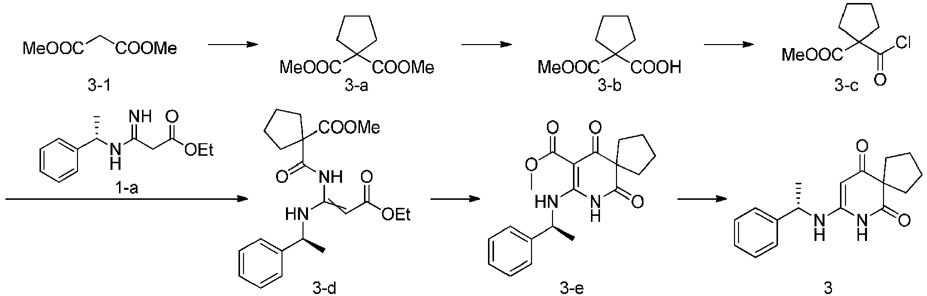

Step A: Under nitrogen atmosphere, to a solution of compound 3-1 (5 g, 37.85 mmol, 4.35 mL, 1 eq) and 1,4-dibromobutane (8.17 g, 37.85 mmol, 4.57 mL, 1 eq) in DMF (50 mL) was added potassium carbonate (10.46 g, 75.69 mmol, 2 eq). The reaction mixture was stirred at 50°C for 16 hours, added with EA (200 mL), washed with water (200 mL × 2), dried over anhydrous sodium sulfate, filtered, and concentrated. The residue was purified by column chromatography (PE: EtOAc = 20:1 to 10:1) to obtain compound 3-a.

Step B: To a solution of compound 3-a (4.5 g, 24.17 mmol, 1 eq) in MeOH (25 mL) and water (25 mL) was added sodium hydroxide (1.06 g, 26.58 mmol, 1.1 eq). The reaction mixture was stirred at 20°C for 16 hours, added with water (30 mL), and extracted with EA (30 mL). After separating the phases, the aqueous phase was added with 1M dilute hydrochloric acid to adjust the pH to around 5, then extracted with EA (30 mL), dried over anhydrous sodium sulfate, filtered, and concentrated to obtain compound 3-b.

Step C: Under nitrogen atmosphere at -20°C, to a solution of compound 3-b (2.1 g, 12.20 mmol, 1 eq) in DCM (30 mL) was added TEA (4.94 g, 48.79 mmol, 6.79 mL, 4 eq) and DMF (44.57 mg, 609.83 µmol, 46.92 µL, 0.05 eq), then oxalyl chloride (2.01 g, 15.86 mmol, 1.39 mL, 1.3 eq) was added thereto. The reaction mixture was stirred at 20°C for 1 hour, then concentrated to obtain compound 3-c.

Step D: Under nitrogen atmosphere at -20°C, to a solution of compound 3-c (2.3 g, 12.07 mmol, 1 eq) in DCM (30 mL) was added TEA (2.44 g, 24.13 mmol, 3.36 mL, 2 eq) and compound 1-a (2.83 g, 12.07 mmol, 1 eq). The reaction mixture was stirred at 0°C for 1 hour, and concentrated. The residue was diluted with EA (30 mL), washed with saturated brine (30 mL), dried over anhydrous sodium sulfate, filtered, and concentrated. The residue was purified by silica gel column chromatography (PE: EtOAc = 20:1 to 5:1) to obtain compound 3-d.

Step E: Under nitrogen atmosphere, to a solution of compound 3-d (0.7 g, 1.80 mmol, 1 eq) in MeOH (4 mL) was added sodium methoxide (1 M, 7.21 mL, 4 eq). The reaction mixture was stirred at 20°C for 16 hours, then added with 1 N dilute hydrochloric acid to adjust the pH to 5, and extracted with EA (20 mL). The organic phase was washed with saturated brine (20 mL), dried over anhydrous sodium sulfate, filtered, and concentrated to obtain compound 3-e.

Step F: To a solution of compound 3-e (0.8 g, 2.34 mmol, 1 eq) in 1,4-dioxane (6 mL) was added hydrochloric acid (4 M, 6 mL, 10.27 eq). The reaction mixture was stirred at 50°C for 16 hours, then diluted with EA (50 mL), added with 1 N sodium hydroxide aqueous solution to adjust the pH to 7. After separating the phases, the organic phase was washed with saturated brine (50 mL × 2), dried over anhydrous sodium sulfate, filtered, and concentrated. The residue was added with MeOH (10 mL), stirred for 20 minutes, filtered. The filter cake was dried under high vacuum to obtain compound 3. ¹H NMR (DMSO-*d₆*, 400 MHz): δ ppm 9.49 (br s, 1H), 7.41 - 7.31 (m, 4H), 7.30 - 7.25 (m, 1H), 6.92 (br d, J=5.1 Hz, 1H), 4.60 (br t, J=6.7 Hz, 1H), 4.42 (s, 1H), 1.91 - 1.80 (m, 4H), 1.72 - 1.65 (m, 4H), 1.43 (d, J=6.8 Hz, 3H); LCMS(ESI) m/z: 285.4(M+1).

### Example 4

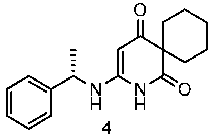

### Synthetic route:

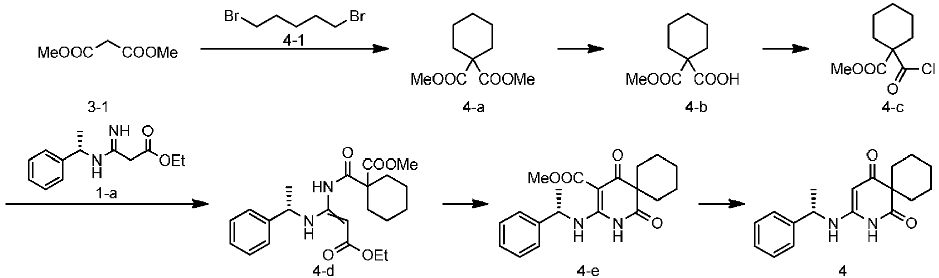

Step A: To a solution of compound 3-1 (14.94 g, 113.07 mmol, 12.99 mL, 1 eq) in DMF (150 mL) was added compound 4-1 (26 g, 113.07 mmol, 15.29 mL, 1 eq) and potassium carbonate (31.25 g, 226.15 mmol, 2 eq). The reaction mixture was stirred at 50°C for 16 hours, then added with EA (150 mL), and washed with water (150 mL). The organic phase was washed with saturated brine (100 mL × 3), dried over anhydrous sodium sulfate, filtered, and concentrated. The residue was purified by column chromatography (PE: EtOAc = 50:1 to 30:1) to obtain compound 4-a.

Step B: To a solution of compound 4-a (6.9 g, 34.46 mmol, 1 eq) in MeOH (40 mL) and water (40 mL) was added sodium hydroxide (1.38 g, 34.46 mmol, 1 eq). The reaction mixture was stirred at 15°C for 16 hours, then added with water (50 mL), and extracted with EA (100 mL). After separating the phases, the aqueous phase was added with 1M dilute hydrochloric acid to adjust the pH to around 5, then extracted with EA (100 mL × 2). The combined organic phases were washed with saturated brine (100 mL), dried over anhydrous sodium sulfate, filtered, and concentrated to obtain compound 4-b.

Step C: At 0°C, to a solution of compound 4-b (4.5 g, 24.17 mmol, 1 eq) in DCM (50 mL) was added TEA (9.78 g, 96.67 mmol, 13.45 mL, 4 eq) and DMF (88.32 mg, 1.21 mmol, 92.97 µL, 0.05 eq), then oxalyl chloride (3.99 g, 31.42 mmol, 2.75 mL, 1.3 eq) was added thereto. The reaction mixture was stirred at 10°C for 1 hour, and concentrated to obtain compound 4-c.

Step D: At 0°C, to a solution of compound 4-c (5 g, 24.43 mmol, 1 eq) in DCM (50 mL) was added TEA (4.94 g, 48.86 mmol, 6.80 mL, 2 eq), then compound 1-a (5.72 g, 24.43 mmol, 1 eq) was added thereto. The reaction mixture was stirred at 0°C for 1 hour, then concentrated. The residue was purified by column chromatography (PE: EtOAc = 30:1 to 10:1) to obtain compound 4-d.

Step E: Under nitrogen atmosphere, to a solution of compound 4-d (2.30 g, 5.71 mmol, 1 eq) in MeOH (15 mL) was added sodium methoxide (1 M, 28.57 mL, 5 eq). The reaction mixture was stirred at 10°C for 16 hours, then stirred at 50°C for 3 hours. The reaction mixture was added with 1 N dilute hydrochloric acid to adjust the pH to around 5, and extracted with EA (50 mL × 2). The combined organic phases were washed with saturated brine (50 mL × 2), dried over anhydrous sodium sulfate, filtered, and concentrated. The residue was purified by silica gel column chromatography (PE: EtOAc = 10:1 to 3:1) to obtain compound 4-e.

Step F: To a solution of compound 4-e (0.7 g, 1.96 mmol, 1 eq) in 1,4-dioxane (10 mL) and THF (2 mL) was added hydrochloric acid (4 M, 10.50 mL, 21.38 eq). The reaction mixture was stirred at 50°C for 40 hours, then added with 1 N sodium hydroxide aqueous solution to adjust the pH to about 7, and extracted with DCM/MeOH at a ratio of 10 to 1 (50 mL × 2). The combined organic phases were washed with saturated brine (30 mL × 2), dried over anhydrous sodium sulfate, filtered, and concentrated. The residue was added with MeOH (10 mL), stirred at 10°C for 30 minutes, and filtered. The filter cake was dried under high vacuum to obtain compound 4. ¹H NMR (DMSO-*d₆*, 400 MHz): δ ppm 9.38 (br s, 1H), 7.41 - 7.22 (m, 5H), 6.84 (br d, *J=* 5.4 Hz, 1H), 4.57 (br t, *J=* 6.8 Hz, 1H), 4.36 (s, 1H), 1.74 - 1.45 (m, 10H), 1.41 (d, *J* = 6.8 Hz, 3H); LCMS(ESI) m/z: 299.2(M+1).

### Example 5

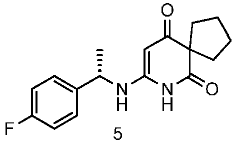

### Synthetic route:

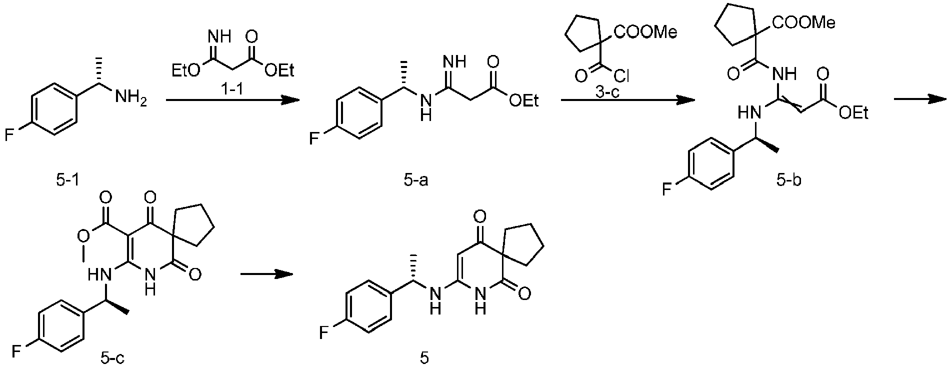

Step A: Under nitrogen atmosphere at 20°C, to a solution of compound 5-1 (1.00 g, 7.19 mmol, 1 eq) and compound 1-1 (1.14 g, 7.19 mmol, 1 eq) in EtOH (15 mL) was added DIEA (1.86 g, 14.37 mmol, 2.50 mL, 2 eq). The reaction mixture was stirred at 20°C for 16 hours, then concentrated to obtain compound 5-a.

Step B: Under nitrogen atmosphere at 20°C, to a solution of compound 5-a (1.81 g, 7.17 mmol, 1 eq) in DCM (30 mL) was added TEA (1.45 g, 14.35 mmol, 2.00 mL, 2 eq), then compound 3-c (1.37 g, 7.17 mmol, 1 eq) was added thereto. The reaction mixture was stirred at 20°C for 16 hours, then concentrated. The residue was diluted with EA (40 mL), washed with water (40 mL), dried over anhydrous sodium sulfate, filtered, and concentrated. The residue was purified by column chromatography (PE: EtOAc = 20:1 to 10:1) to obtain compound 5-b.

Step C: Under nitrogen atmosphere at 20°C, to a solution of compound 5-b (600 mg, 1.48 mmol, 1 eq) in MeOH (6 mL) was added sodium methoxide (1 M, 5.90 mL, 4 eq). The reaction mixture was stirred at 40°C for 16 hours, added to water (40 mL), then extracted with EA (40 mL × 2). The combined organic phases were washed with saturated brine (40 mL), dried over anhydrous sodium sulfate, filtered, and concentrated. The residue was purified by silica gel column chromatography (PE: EtOAc = 5:1 to 1:1) to obtain compound 5-c.

Step D: At 20°C, to a solution of compound 5-c (550 mg, 1.53 mmol, 1 eq) in 1,4-dioxane (4 mL) was added hydrochloric acid (4 M, 3.82 mL, 10 eq). The reaction mixture was stirred at 40°C for 16 hours, added with EA (40 mL), then added with 1 N sodium hydroxide aqueous solution to adjust the pH to around 7. The phases were separated. The aqueous phase was extracted with EA (40 mL). The combined organic phases were washed with saturated brine (50 mL), dried over anhydrous sodium sulfate, filtered, and concentrated. The residue was added with MeOH (5 mL), stirred, and filtered. The filter cake was dried under high vacuum to obtain compound 5. ¹H NMR (DMSO-d6, 400 MHz):δ ppm 9.48 (br s, 1H), 7.38 (dd, J=5.6, 8.6 Hz, 2H), 7.19 (t, J=8.8 Hz, 2H), 6.91 (br d, J=6.0 Hz, 1H), 4.62 (br t, J=6.7 Hz, 1H), 4.41 (s, 1H), 1.96 - 1.78 (m, 4H), 1.76 - 1.61 (m, 4H), 1.42 (d, J=6.8 Hz, 3H); LCMS(ESI) m/z: 302.8(M+ 1).

### Example 6

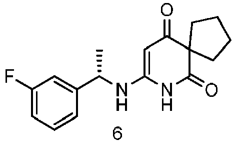

### Synthetic route:

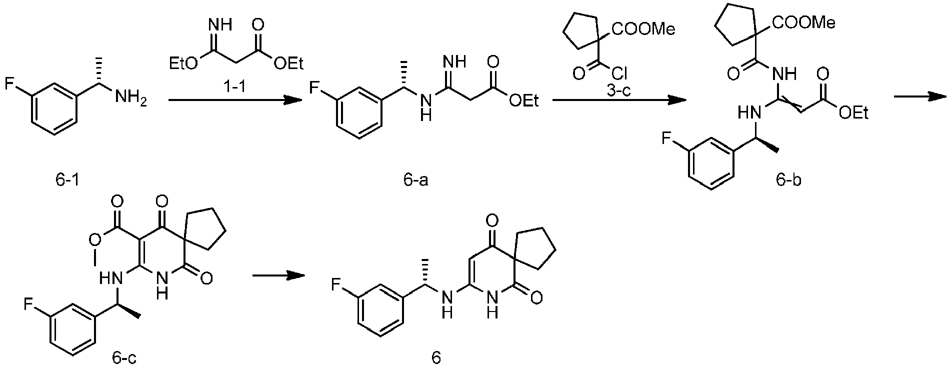

Step A: Under nitrogen atmosphere at 20°C, to a solution of compound 6-1 (1.00 g, 7.19 mmol, 1 eq) and compound 1-1 (1.14 g, 7.19 mmol, 1 eq) in EtOH (15 mL) was added DIEA (1.86 g, 14.37 mmol, 2.50 mL, 2 eq). The reaction mixture was stirred at 20°C for 16 hours, then concentrated to obtain compound 6-a.

Step B: Under nitrogen atmosphere at 20°C, to a solution of compound 6-a (1.81 g, 7.17 mmol, 1 eq) in DCM (30 mL) was added TEA (1.45 g, 14.35 mmol, 2.00 mL, 2 eq), then compound 3-c (1.37 g, 7.17 mmol, 1 eq) was added thereto. The reaction mixture was stirred at 20°C for 16 hours, then concentrated. The residue was diluted with EA (40 mL), washed with water (40 mL), dried over anhydrous sodium sulfate, filtered, and concentrated. The residue was purified by column chromatography (PE: EtOAc = 20:1 to 10:1) to obtain compound 6-b.

Step C: Under nitrogen atmosphere at 20°C, to a solution of compound 6-b (750.00 mg, 1.85 mmol, 1 eq) in MeOH (8 mL) was added sodium methoxide (1 M, 8 mL, 4.34 eq). The reaction mixture was stirred at 40°C for 16 hours. After cooling to room temperature, the mixture was added with EA (30 mL), added with 1 N hydrochloric acid to adjust the pH to around 7. The phases were separated. The aqueous phase was extracted with EA (40 mL × 2). The combined organic phases were washed with saturated brine (40 mL), dried over anhydrous sodium sulfate, filtered, and concentrated. The residue was purified by column chromatography (PE: EtOAc = 5:1 to 1:1) to obtain compound 6-c.

Step D: At 20°C, to a solution of compound 6-c (550 mg, 1.53 mmol, 1 eq) in 1,4-dioxane (4 mL) was added hydrochloric acid (4 M, 3.82 mL, 10 eq). The reaction mixture was stirred at 40°C for 16 hours, added with water (40 mL), then added with saturated sodium bicarbonate aqueous solution to adjust the pH to around 7, and extracted with EA (40 mL × 2). The combined organic phases were washed with saturated brine (40 mL), dried over anhydrous sodium sulfate, filtered, and concentrated. The residue was added with MeOH (6 mL), stirred for 15 minutes, and filtered. The filter cake was dried under high vacuum to obtain compound 6. ¹H NMR (DMSO-*d₆*, 400 MHz):δ ppm 9.51 (br s, 1H), 7.49 - 7.35 (m, 1H), 7.19 (br d, J=7.7 Hz, 2H), 7.09 (dt, *J*=1.6, 8.2 Hz, 1H), 6.94 (br d, J=6.1 Hz, 1H), 4.63 (br t, J=6.7 Hz, 1H), 4.40 (s, 1H), 1.90 - 1.79 (m, 4H), 1.74 - 1.63 (m, 4H), 1.42 (d, J=6.7 Hz, 3H); LCMS(ESI) m/z: 302.8(M+1).

### Example 7

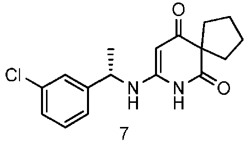

### Synthetic route:

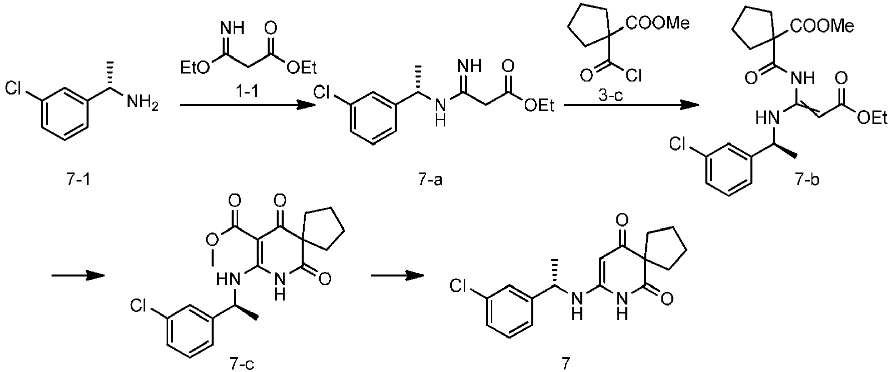

Step A: Under nitrogen atmosphere at 20°C, to a solution of compound 7-1 (0.76 g, 4.88 mmol, 1 eq) and compound 1-1 (777.38 mg, 4.88 mmol, 1 eq) in EtOH (15 mL) was added DIEA (1.26 g, 9.77 mmol, 1.70 mL, 2 eq). The reaction mixture was stirred at 20°C for 16 hours, then concentrated to obtain compound 7-a.

Step B: Under nitrogen atmosphere at 20°C, to a solution of compound 7-a (1.31 g, 4.87 mmol, 1 eq) in DCM (30 mL) was added TEA (986.53 mg, 9.75 mmol, 1.36 mL, 2 eq), then compound 3-c (1.23 g, 6.45 mmol, 1.32 eq) was added thereto. The reaction mixture was stirred at 20°C for 16 hours, then concentrated. The residue was diluted with EA (40 mL), washed with water (40 mL), dried over anhydrous sodium sulfate, filtered, and concentrated. The residue was purified by column chromatography (PE: EtOAc = 20:1 to 10:1) to obtain compound 7-b.

Step C: Under nitrogen atmosphere at 20°C, to a solution of compound 7-b (750 mg, 1.77 mmol, 1 eq) in MeOH (8 mL) was added sodium methoxide (1 M, 8 mL, 4.51 eq). The reaction mixture was stirred at 40°C for 16 hours. After cooling to room temperature, the mixture was added with EA (30 mL), then added with 1 N hydrochloric acid to adjust the pH to around 7. The phases were separated. The aqueous phase was extracted with EA (60 mL × 2). The combined organic phases were washed with saturated brine (60 mL), dried over anhydrous sodium sulfate, filtered, and concentrated. The residue was purified by column chromatography (PE: EtOAc = 4:1 to 1:1) to obtain compound 7-c.

Step D: At 20°C, to a solution of compound 7-c (575.11 mg, 1.53 mmol, 1 eq) in 1,4-dioxane (4 mL) was added hydrochloric acid (4 M, 3.82 mL, 10 eq). The reaction mixture was stirred at 40°C for 16 hours, added with water (40 mL), then added with saturated sodium bicarbonate aqueous solution to adjust the pH to around 7, and extracted with EA (40 mL × 2). The combined organic phases were washed with saturated brine (40 mL), dried over anhydrous sodium sulfate, filtered, and concentrated. The residue was added with MeOH (4 mL), stirred for 15 minutes, and filtered. The filter cake was dried under high vacuum to obtain compound 7. ¹H NMR (DMSO-*d₆*, 400 MHz) δ ppm 9.51 (br s, 1H), 7.48 - 7.37 (m, 2H), 7.36 - 7.27 (m, 2H), 6.95 (br d, J=5.1 Hz, 1H), 4.63 (br t, J=6.7 Hz, 1H), 4.41 (s, 1H), 1.90 - 1.80 (m, 4H), 1.72 - 1.64 (m, 4H), 1.42 (d, J=6.8 Hz, 3H); LCMS(ESI) m/z: 318.8(M+1).

### Example 8

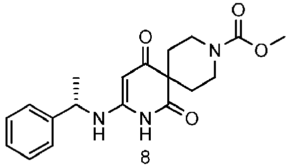

### Synthetic route:

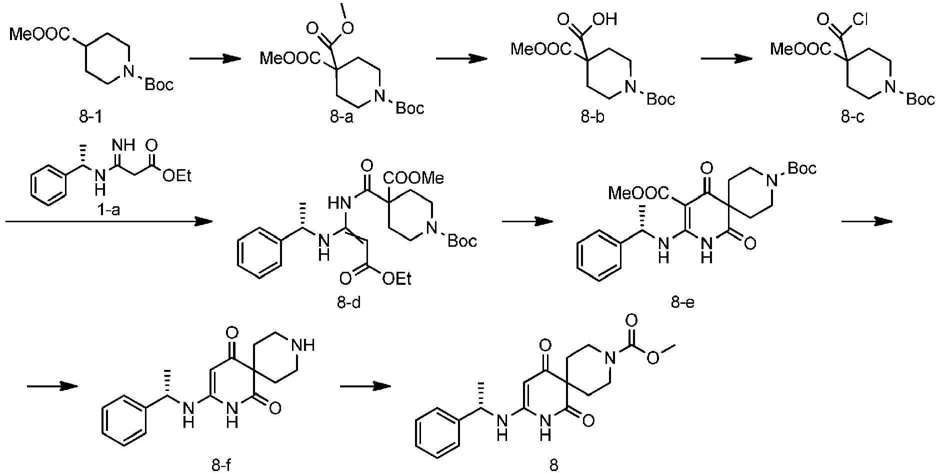

Step A: Under nitrogen atmosphere at -78°C, to a solution of compound 8-1 (20 g, 82.20 mmol, 1 eq) in THF (200 mL) was dropwise added LDA (2 M, 49.32 mL, 1.2 eq). The reaction mixture was stirred at -78°C for 1 hour, then was added with methyl chloroformate (8.54 g, 90.42 mmol, 7.00 mL, 1.1 eq). The reaction mixture was slowly warmed to 20°C, stirred for 4 hours, then quenched with saturated ammonium chloride aqueous solution (600 mL), and extracted with EA (600 mL). The organic phase was washed with saturated brine (200 mL × 2), dried over anhydrous sodium sulfate, filtered, and concentrated to obtain compound 8-a.

Step B: To a solution of compound 8-a (27 g, 89.60 mmol, 1 eq) in MeOH (200 mL) and water (200 mL) was added sodium hydroxide (3.58 g, 89.60 mmol, 1 eq). The reaction mixture was stirred at 15°C for 16 hours, then added with water (200 mL), and extracted with EA (200 mL). After separating the phases, the aqueous phase was added with 1 M dilute hydrochloric acid to adjust the pH to around 5, then extracted with EA (300 mL × 2). The combined organic phases were washed with saturated brine (200 mL), dried over anhydrous sodium sulfate, filtered, and concentrated to obtain compound 8-b.

Step C: Under nitrogen atmosphere at 0°C, to a solution of compound 8-b (23 g, 80.05 mmol, 1 eq) in DCM (200 mL) was added TEA (32.40 g, 320.21 mmol, 44.57 mL, 4 eq) and DMF (292.56 mg, 4.00 mmol, 307.95 µL, 0.05 eq), then oxalyl chloride (13.21 g, 104.07 mmol, 9.11 mL, 1.3 eq) was added thereto. The reaction mixture was stirred at 10°C for 1 hour, then concentrated to obtain compound 8-c.

Step D: Under nitrogen atmosphere at 0°C, to a solution of compound 1-a (23.38 g) in DCM (200 mL) was added TEA (23.83 g, 235.47 mmol, 32.78 mL, 3 eq), and a solution of compound 8-c (24 g, 78.49 mmol, 1 eq) in DCM (200 mL) was added thereto. The reaction mixture was stirred at 10°C for 16 hours, then concentrated. The residue was purified by silica gel column chromatography (PE: EtOAc = 10:1 to 5:1) to obtain compound 8-d.

Step E: Under nitrogen atmosphere, to a solution of compound 8-d (13.4 g, 26.61 mmol, 1 eq) in MeOH (130 mL) was added sodium methoxide (1 M, 130 mL, 4.89 eq). The reaction mixture was stirred at 50°C for 3 hours, then concentrated. The residue was added with 1 M dilute hydrochloric acid to adjust the pH to between 5 and 6, then extracted with EA (200 mL × 2). The combined organic phases were washed with saturated brine (200 mL × 2), dried over anhydrous sodium sulfate, filtered, and concentrated. The residue was purified by silica gel column chromatography (PE: EtOAc = 3:1 to 1:1) to obtain compound 8-e.

Step F: To a solution of compound 8-e (7.5 g, 16.39 mmol, 1 eq) in 1,4-dioxane (150 mL) was added hydrochloric acid (4 M, 150 mL, 36.60 eq). The reaction mixture was stirred at 80°C for 16 hours. After cooling, the reaction mixture was added with 2 N sodium hydroxide aqueous solution to adjust the pH to between 8 and 9, then extracted with EA /iPrOH at a ratio of 7 to 1 (200 mL × 4). The combined organic phases were dried over anhydrous sodium sulfate, filtered, and concentrated to obtain compound 8-f.

Step G: Under nitrogen atmosphere at 0°C, to a solution of compound 8-f (0.15 g, 501.06 µmol, 1 eq) in DCM (3 mL) was added DIEA (194.27 mg, 1.50 mmol, 261.83 µL, 3 eq), then a solution of methyl chloroformate (49.72 mg, 526.11 µmol, 40.75 µL, 1.05 eq) in DCM (1 mL) was added thereto. The reaction mixture was stirred at 0°C for 1 hour, and filtered. The filtrate was concentrated. The residue was purified by preparative HPLC [mobile phase: water (0.05% ammonium water)-ACN; gradient: 15% to 45% ACN] to obtain compound 8. ¹H NMR (DMSO-*d₆*, 400 MHz): δ ppm 9.80 - 9.51 (m, 1H), 7.40 - 7.31 (m, 4H), 7.30 - 7.23 (m, 1H), 7.11 - 6.97 (m, 1H), 4.60 (br s, 1H), 4.43 (s, 1H), 3.73 - 3.64 (m, 2H), 3.58 (s, 3H), 3.48 - 3.34 (m, 2H), 1.82 - 1.70 (m, 2H), 1.69 - 1.55 (m, 2H), 1.42 (d, J= 6.8 Hz, 3H); LCMS(ESI) m/z: 358.2(M+1).

### Example 9

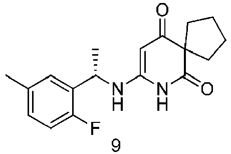

### Synthetic route:

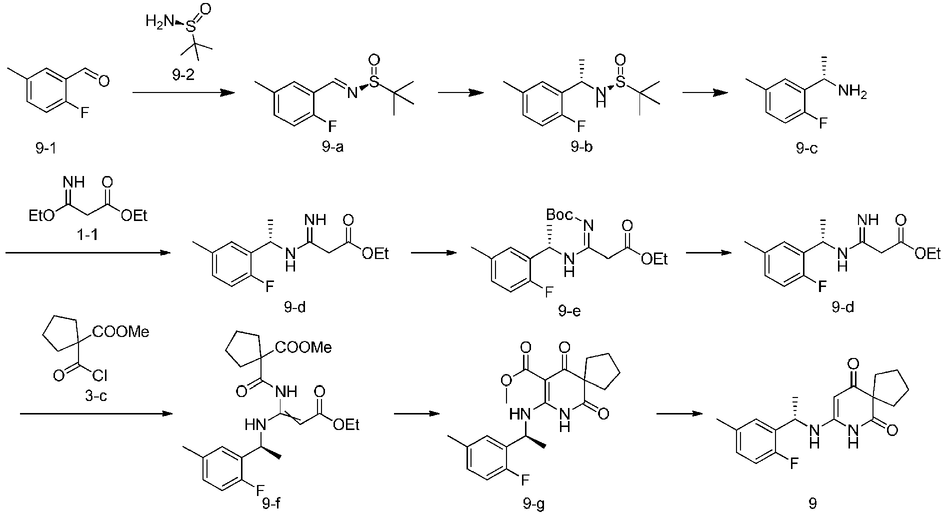

Step A: Under nitrogen atmosphere, to a solution of compound 9-1 (10 g, 72.39 mmol, 1 eq) and 9-2 (9.21 g, 76.01 mmol, 1.05 eq) in DCM (200 mL) was added cesium carbonate (35.38 g, 108.59 mmol, 1.5 eq). The reaction mixture was stirred at 15°C for 16 hours, and filtered. The filtrate was concentrated to obtain compound 9-a.

Step B: Under nitrogen atmosphere at -78°C, to a solution of compound 9-a (9 g, 37.29 mmol, 1 eq) in THF (100 mL) was slowly added methylmagnesium bromide (3 M, 24.86 mL, 2 eq). The reaction mixture was stirred at -78°C for 1 hour, then was slowly added dropwise to a saturated ammonium chloride aqueous solution (800 mL). The mixture was extracted with EA (200 mL × 2). The combined organic phases were washed with saturated brine (100 mL × 3), dried over anhydrous sodium sulfate, filtered, and concentrated. The residue was purified by column chromatography (PE: EtOAc = 10:1 to 3:1) to obtain compound 9-b.

Step C: To a solution of compound 9-b (12 g, 46.63 mmol, 1 eq) in MeOH (100 mL) was added HCl/MeOH (4 M, 100 mL, 8.58 eq). The reaction mixture was stirred at 20°C for 2 hours, and concentrated to obtain the hydrochloride of compound 9-c.

Step D: To a solution of the hydrochloride of compound 9-c (7.5 g) in EtOH (100 mL) was added compound 1-1 (9.58 g, 48.96 mmol, 1 eq, HCl) and DIEA (25.31 g, 195.83 mmol, 34.11 mL, 4 eq). The reaction mixture was stirred at 20°C for 16 hours, and concentrated to obtain compound 9-d.

Step E: To a solution of compound 9-d (15 g, 56.33 mmol, 1 eq) in DCM (150 mL) was added Boc₂O (18.44 g, 84.49 mmol, 19.41 mL, 1.5 eq) and TEA (17.10 g, 168.98 mmol, 23.52 mL, 3 eq). The reaction mixture was stirred at 15°C for 16 hours, then concentrated under reduced pressure. The residue was purified by column chromatography (PE: EtOAc = 15:1) to obtain compound 9-e.

Step F: To a solution of compound 9-e (8.00 g, 21.83 mmol, 1 eq) in EtOAc (50 mL) was added HCl/EtOAc (4 M, 51.61 mL, 9.46 eq). The reaction mixture was stirred at 20°C for 16 hours, and concentrated to obtain the hydrochloride of compound 9-d.

Step G: Under nitrogen atmosphere at -20°C, to a solution of the hydrochloride of compound 9-d (8.80 g) in DCM (60 mL) was added TEA (10.03 g, 99.13 mmol, 13.80 mL, 3 eq) and compound 3-c (2.83 g, 12.07 mmol, 1 eq). The reaction mixture was stirred at -20°C for 1 hour, then warmed to 20°C, stirred for 15 hours, and concentrated. The residue was purified by column chromatography (PE: EtOAc = 30:1 to 10:1) to obtain compound 9-f.

Step H: Under nitrogen atmosphere, to a solution of compound 9-f(3.5 g, 8.32 mmol, 1 eq) in MeOH (40 mL) was added sodium methoxide (1 M, 41.62 mL, 5 eq). The reaction mixture was stirred at 50°C for 16 hours, then added with 1 N dilute hydrochloric acid to adjust the pH to around 5, then extracted with EA (100 mL × 2). The combined organic phases were washed with saturated brine (50 mL × 2), dried over anhydrous sodium sulfate, filtered, and concentrated to obtain compound 9-g.

Step J: To a solution of compound 9-g (3 g, 8.01 mmol, 1 eq) in 1,4-dioxane (60 mL) was added hydrochloric acid (4 M, 60 mL, 29.95 eq). The reaction mixture was stirred at 70°C for 16 hours, added with 2 N sodium hydroxide aqueous solution to adjust the pH to between 8 and 9, then filtered. The filter cake was first purified by silica gel column chromatography (DCM: MeOH = 1:0 to 10:1), then added with MTBE (50 mL), stirred for 1 hour, and filtered. The filter cake was dried under high vacuum to obtain compound 9. ¹H NMR (DMSO-*d₆*, 400 MHz): δ ppm 9.52 (br s, 1 H) 7.19 (br d, J=7.5 Hz, 1 H) 7.06 - 7.15 (m, 2 H) 6.91 (br d, J=6.8 Hz, 1 H) 4.66 - 4.76 (m, 1 H) 4.40 (s, 1 H) 2.28 (s, 3 H) 1.81 - 1.90 (m, 4 H) 1.64 - 1.72 (m, 4 H) 1.45 (d, J=6.8 Hz, 3 H); LCMS(ESI) m/z: 317.2(M+1).

### Example 10

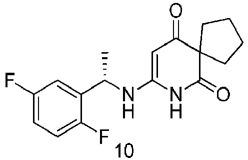

### Synthetic route:

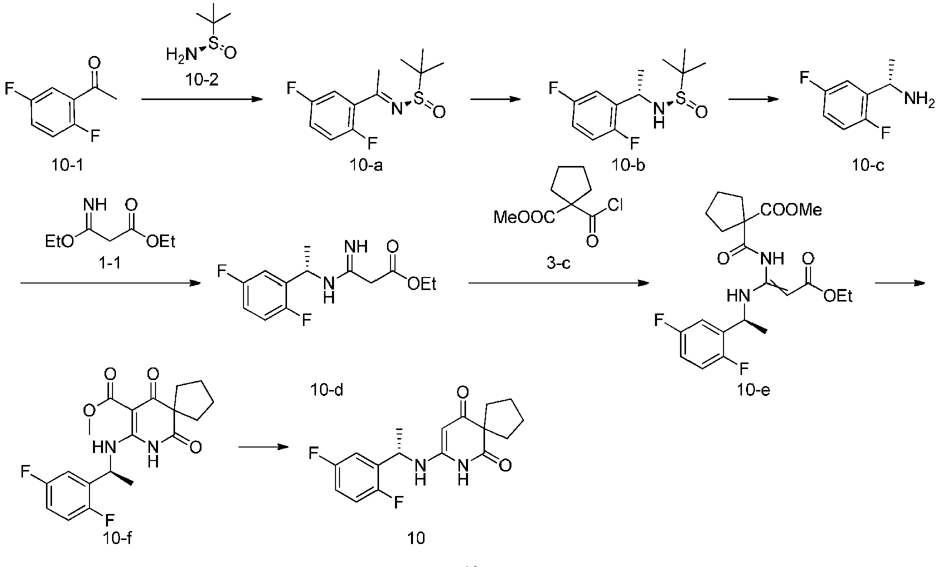

Step A: At 20°C, to a solution of compound 10-1 (5 g, 32.02 mmol, 4.07 mL, 1 eq) in THF (50 mL) was added compound 10-2 (4.66 g, 38.43 mmol, 1.2 eq) and titanium(IV) ethoxide (21.92 g, 96.07 mmol, 19.92 mL, 3 eq). The reaction mixture was stirred at 60°C for 16 hours, then added with ethyl acetate (100 mL). After cooling to 0°C, the mixture was slowly added with water (20 mL), stirred for 0.5 hours, and filtered. The filtrate was washed with saturated brine (50 mL × 3), dried over anhydrous sodium sulfate, filtered, and concentrated to obtain compound 10-a.

Step B: Under nitrogen atmosphere at -78°C, to a solution of compound 10-a (9 g, 34.71 mmol, 1 eq) in THF (100 mL) was slowly added L-selectridee (1 M, 41.65 mL, 1.2 eq). The reaction mixture was stirred at -78°C for 2 hours, then was slowly added to a saturated ammonium chloride aqueous solution (100 mL). The mixture was extracted with EA (100 mL × 2). The combined organic phases were washed with saturated brine (100 mL × 3), dried over anhydrous sodium sulfate, filtered, and concentrated. The residue was purified by column chromatography (PE: EtOAc = 5:1 to 3:1) to obtain compound 10-b.

Step C: At 20°C, to a solution of compound 10-b (6.6 g) in MeOH (50 mL) was added HCl/MeOH (200 mmol, 50 mL, 7.92 eq). The reaction mixture was stirred for 16 hours, and concentrated to obtain the hydrochloride of compound 10-c.

Step D: At 20°C, to a solution of the hydrochloride of compound 10-c (1 g) in EtOH (10 mL) was added compound 1-1 (1.51 g, 7.74 mmol, 1.5eq, HCL) and DIEA (4.00 g, 30.96 mmol, 5.40 mL, 6 eq). The reaction mixture was stirred at 20°C for 16 hours, then concentrated to obtain compound 10-d.

Step E: Under nitrogen atmosphere at -20°C, to a solution of compound 10-d (1.54 g, 8.08 mmol, 1 eq) in DCM (15 mL) was added TEA (2.45 g, 24.24 mmol, 3.37 mL, 3 eq), then a solution of compound 3-c (1.39 g, 5.14 mmol, 6.37e⁻¹ eq) in DCM (15 mL) was added thereto. The reaction mixture was stirred at 20°C for 16 hours, then concentrated. The residue was diluted with EA (30 mL), washed with saturated brine (30 mL), dried over anhydrous sodium sulfate, filtered, and concentrated. The residue was purified by column chromatography (PE: EtOAc = 10:1 to 5:1) to obtain compound 10-e.

Step F: Under nitrogen atmosphere, to a solution of compound 10-e (0.648 g, 1.53 mmol, 1 eq) in MeOH (7.6 mL) was added sodium methoxide (1 M, 6.43ml, 1 eq). The reaction mixture was stirred at 20°C for 16 hours, added with 1 M dilute hydrochloric acid to adjust the pH to around 5, and then extracted with EA (20 mL). The organic phase was washed with saturated brine (20 mL), dried over anhydrous sodium sulfate, filtered, and concentrated to obtain compound 10-f.

Step G: To a solution of compound 10-f(348 mg, 919.74 µmol, 1 eq) in 1,4-dioxane (4 mL) was added hydrochloric acid (4 M, 4 mL, 17.40 eq). The reaction mixture was stirred at 50°C for 16 hours, added with EA (30 mL), and then added with 1 M sodium hydroxide aqueous solution to adjust the pH to around 8. After separating the phases, the organic phase was washed with saturated brine (50 mL × 2), dried over anhydrous sodium sulfate, filtered, and concentrated. The residue was added with MeOH (10 mL), stirred for 20 minutes, and filtered. The filter cake was dried under high vacuum to obtain compound 10. ¹H NMR (DMSO-*d₆*, 400 MHz): δ ppm 9.60 - 9.47 (m, 1H), 7.34 - 7.26 (m, 2H), 7.25 - 7.16 (m, 1H), 6.96 (br d, *J=* 6.0 Hz, 1H), 4.82 - 4.71 (m, 1H), 4.44 - 4.34 (m, 1H), 1.93 - 1.81 (m, 4H), 1.70 (br s, 4H), 1.48 (br d, *J* = 6.4 Hz, 3H); LCMS(ESI) m/z: 321.2(M+1).

### Example 11

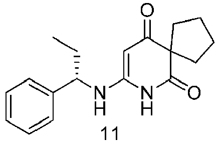

### Synthetic route:

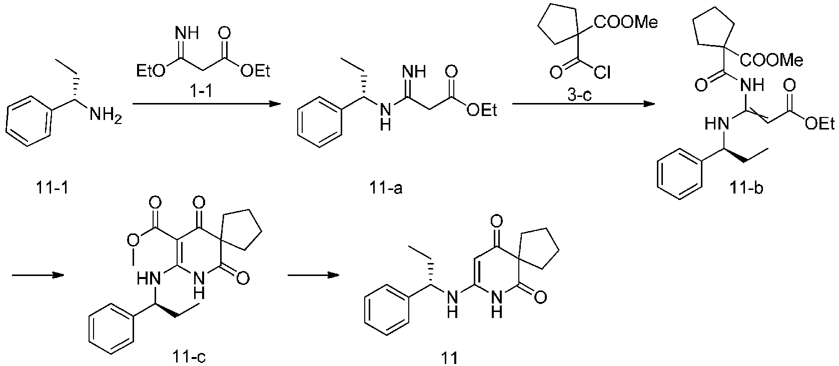

Step A: At 20°C, to a solution of compound 11-1 (1g, 7.40 mmol, 1.06 mL, 1 eq) in EtOH (15 mL) was added compound 1-1 (1.18 g, 7.40 mmol, 1.0 eq, HCl) and DIEA (2.87 g, 22.19 mmol, 3.86 mL, 3 eq). The reaction mixture was stirred at 20°C for 12 hours, then concentrated to obtain compound 11-a.

Step B: Under nitrogen atmosphere at -20°C, to a solution of compound 11-a (2.44 g, 9.83 mmol, 6.91e⁻¹ eq) in DCM (15 mL) was added TEA (4.32 g, 42.65 mmol, 5.94 mL, 3 eq), then a solution of compound 3-c (2.71 g, 14.22 mmol, 6.37e⁻¹ eq) in DCM (15 mL) was added thereto. The reaction solution was stirred at 20°C for 16 hours, and concentrated. The residue was diluted with EA (30 mL), washed with saturated brine (30 mL × 3), dried over anhydrous sodium sulfate, filtered, and concentrated. The residue was purified by column chromatography (PE: EtOAc = 10:1 to 8:1) to obtain compound 11-b.

Step C: Under nitrogen atmosphere, to a solution of compound 11-b (0.743 g, 1.47 mmol, 1 eq) in MeOH (9.2 mL) was added sodium methoxide (1 M, 7.37 mL, 5 eq). The reaction mixture was stirred at 20°C for 16 hours, added with 1 M dilute hydrochloric acid to adjust the pH to around 5, and extracted with EA (20 mL × 3). The combined organic phases were washed with saturated brine (20 mL × 3), dried over anhydrous sodium sulfate, filtered, and concentrated to obtain compound 11-c.

Step D: To a solution of compound 11-c (399 mg, 1.12 mmol, 1 eq) in 1,4-dioxane (4 mL) was added hydrochloric acid (4 M, 4 mL, 17.40 eq). The reaction mixture was stirred at 50°C for 16 hours, then diluted with EA (30 mL), added with 2 M sodium hydroxide aqueous solution to adjust the pH to around 8, and filtered. The filter cake was added with MeOH (10 mL), stirred for 1 hour, and filtered, and the filter cake was dried under high vacuum to obtain compound 11. ¹H NMR (DMSO-*d₆*, 400 MHz): δ ppm 9.52 (br s, 1H), 7.41 - 7.25 (m, 5H), 6.97 - 6.85 (m, 1H), 4.42 (s, 1H), 4.40 - 4.29 (m, 1H), 1.90 - 1.84 (m, 2H), 1.83 - 1.63 (m, 8H), 0.87 (t, *J=* 7.2 Hz, 3H); LCMS(ESI) m/z: 299.1(M+1).

### Example 12

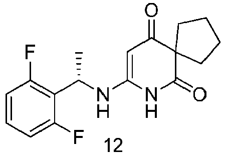

### Synthetic route:

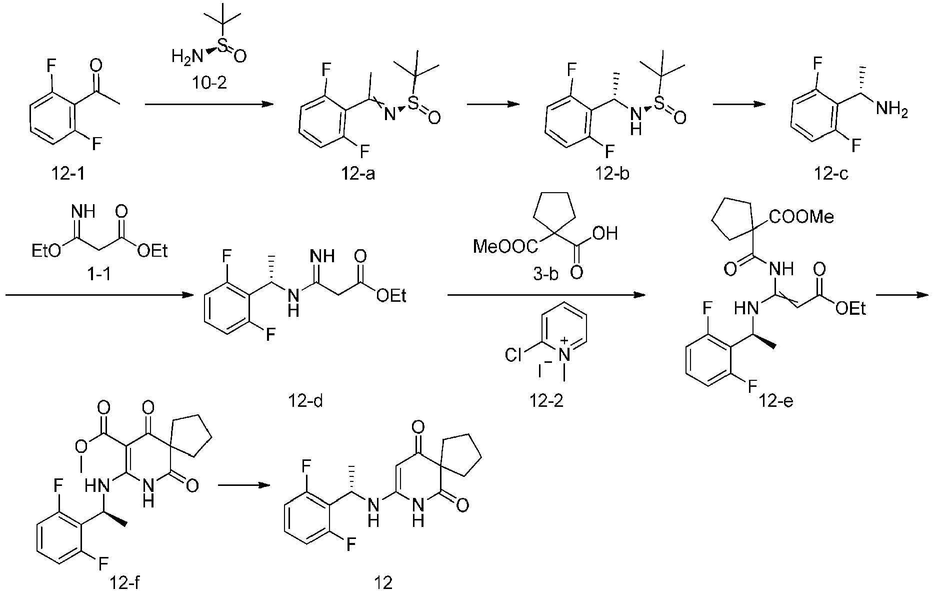

Step A: At 20°C, to a solution of compound 12-1 (10 g, 64.05 mmol, 8.33 mL, 1 eq) in THF (100 mL) was added compound 10-2 (17.08 g, 140.91 mmol, 2.2 eq) and titanium(IV) ethoxide (43.83 g, 192.15 mmol, 39.85 mL, 3 eq). The reaction mixture was stirred at 60°C for 16 hours. After cooling to 0°C, the reaction mixture was added with EA (100 mL), slowly added with water (30 mL), stirred for 0.5 hours, and filtered. The filtrate was washed with saturated brine (30 mL × 3), dried over anhydrous sodium sulfate, filtered, and concentrated to obtain compound 12-a.

Step B: Under nitrogen atmosphere at -78°C, to a solution of compound 12-a (5.45 g, 21.01 mmol, 1 eq) in THF (50 mL) was slowly added L-selectride (1 M, 25.21 mL, 1.2 eq). The reaction mixture was stirred at 20°C for 2 hours, then was slowly added to a saturated ammonium chloride aqueous solution (40 mL) at 0°C. The mixture was extracted with EA (30 mL × 2). The combined organic phases were washed with saturated brine (30 mL × 3), dried over anhydrous sodium sulfate, filtered, and concentrated. The residue was purified by column chromatography (PE: EtOAc = 8:1 to 1:1) to obtain compound 12-b.

Step C: At 20°C, to a solution of compound 12-b (3.14 g, 12.00 mmol, 1 eq) in MeOH (30 mL) was added HCl/MeOH (4 M, 30 mL, 10.00 eq). The reaction mixture was stirred for 16 hours, then concentrated. The residue was added with EA (20 mL), stirred for 0.5 hours, and filtered. The filter cake was dried under high vacuum to obtain the hydrochloride of compound 12-c.

Step D: Under nitrogen atmosphere at 20°C, to a solution of the hydrochloride of compound 12-c (1.84 g) in EtOH (20 mL) was added compound 1-1 (1.87 g, 11.72 mmol, 1 eq) and DIEA (4.54 g, 35.16 mmol, 6.12 mL, 3 eq). The reaction mixture was stirred at 20°C for 12 hours, then concentrated to obtain compound 12-d.

Step E: Under nitrogen atmosphere, to a solution of compound 12-d (9.11 g, 33.71 mmol, 1 eq) in THF (50 mL) was added compound 3-b (5.80 g, 33.71 mmol, 1 eq) and DIEA (6.53 g, 50.56 mmol, 8.81 mL, 1.5 eq), then compound 12-2 (12.92 g, 50.56 mmol, 1.5 eq) was added thereto. The reaction mixture was stirred at 20°C for 1 hour, then concentrated. The residue was diluted with water (50 mL), and extracted with EA (20 mL × 3). The combined organic phases were washed with saturated brine (20 mL × 3), dried over anhydrous sodium sulfate, filtered, and concentrated. The residue was purified by silica gel column chromatography (PE: EtOAc = 10:1) to obtain compound 12-e.

Step F: Under nitrogen atmosphere, to a solution of compound 12-e (3.78 g, 8.91 mmol, 1 eq) in MeOH (44.56 mL) was added sodium methoxide (1 M, 44.56 mL, 5 eq). The reaction mixture was stirred at 50°C for 16 hours, added with 1 M dilute hydrochloric acid to adjust the pH to around 5, and extracted with EA (50 mL × 2). The combined organic phases were washed with saturated brine (50 mL × 2), dried over anhydrous sodium sulfate, filtered, and concentrated. The residue was purified by column chromatography (PE: EtOAc = 5:1 to 2:1) to obtain compound 12-f.

Step G: To a solution of compound 12-f (389 mg, 1.03 mmol, 1 eq) in 1,4-dioxane (4 mL) was added hydrochloric acid (4 M, 3.91 mL, 15.21 eq). The reaction mixture was stirred at 60°C for 16 hours, then added with 2 M sodium hydroxide aqueous solution to adjust the pH to around 8, and filtered. The filter cake was added with MTBE (5 mL), stirred for 1 hour, and filtered. The resulting filter cake was dried to obtain compound 12. ¹H NMR (DMSO-*d₆,* 400 MHz): δ ppm 9.52 (br s, 1H), 7.47 - 7.35 (m, 1H), 7.14 (br t, *J* = 8.8 Hz, 2H), 7.01 - 6.91 (m, 1H), 4.91 - 4.79 (m, 1H), 4.44 (s, 1H), 1.91 - 1.75 (m, 4H), 1.74 - 1.64 (m, 4H), 1.57 (d, *J=* 6.8 Hz, 3H); LCMS(ESI) m/z: 321.1(M+1).

### Example 13

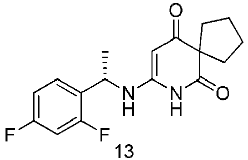

### Synthetic route:

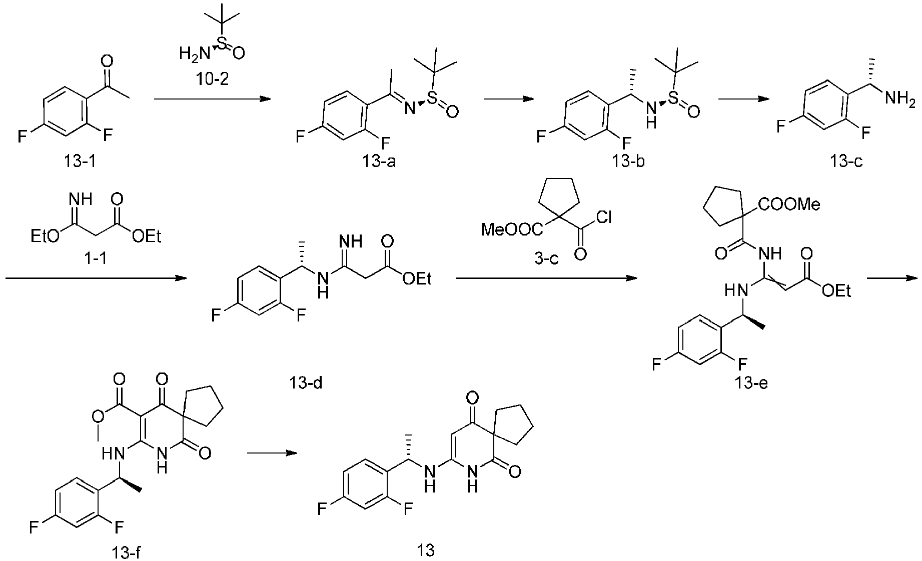

Step A: At 20°C, to a solution of compound 13-1 (3 g, 19.21 mmol, 2.42 mL, 1 eq) in THF (30 mL) was added compound 10-2 (4.66 g, 38.43 mmol, 2 eq) and titanium(IV) ethoxide (13.15 g, 57.64 mmol, 11.95 mL, 3 eq). The reaction mixture was stirred at 60°C for 16 hours, and EA (60 mL) was added thereto. After cooling to 0°C, the mixture was slowly added with water (10 mL), stirred for 0.5 hours, and filtered. The filtrate was washed with saturated brine (30 mL × 3), dried over anhydrous sodium sulfate, filtered, and concentrated to obtain compound 13-a.

Step B: Under nitrogen atmosphere at -78°C, to a solution of compound 13-a (3 g, 11.57 mmol, 1 eq) in THF (20 mL) was slowly added dropwise L-selectride (1 M, 13.88 mL, 1.2 eq). The reaction mixture was stirred at -78°C for 2 hours, then was slowly added to a saturated ammonium chloride aqueous solution (20 mL) at 0°C. The mixture was extracted with EA (20 mL × 2). The combined organic phases were washed with saturated brine (20 mL × 3), dried over anhydrous sodium sulfate, filtered, and concentrated. The residue was purified by column chromatography (PE: EtOAc = 5:1 to 2:1) to obtain compound 13-b.

Step C: To a solution of compound 13-b (1.26 g, 4.81 mmol, 1 eq) in MeOH (15 mL) was added HCl/MeOH (4 M, 15 mL, 12.48 eq). The reaction mixture was stirred at 20°C for 16 hours, and concentrated. The residue was added with EA (20 mL), stirred for 0.5 hours, and filtered. The filter cake was dried under vacuum to obtain the hydrochloride of compound 13-c.

Step D: Under nitrogen atmosphere at 20°C, to a solution of the hydrochloride of compound 13-c (0.553 g) in EtOH (5 mL) was added compound 1-1 (560.12 mg, 3.52 mmol, 1 eq) and DIEA (1.36 g, 10.56 mmol, 1.84 mL, 3 eq). The reaction mixture was stirred at 20°C for 12 hours, then concentrated to obtain compound 13-d.

Step E: Under nitrogen atmosphere at -20°C, to a solution of compound 3-c (1.05 g, 5.51 mmol, 1 eq) in DCM (15 mL) was added TEA (1.67 g, 16.52 mmol, 2.30 mL, 3 eq), then compound 13-d (950.02 mg, 3.52 mmol) was added thereto. The reaction mixture was stirred at 20°C for 16 hours, then concentrated. The residue was diluted with water (50 mL), and extracted with EA (20 mL × 3). The combined organic phases were washed with saturated brine (20 mL × 3), dried over anhydrous sodium sulfate, filtered, and concentrated. The residue was purified by column chromatography (PE: EtOAc = 8:1 to 1:1) to obtain compound 13-e.

Step F: Under nitrogen atmosphere, to a solution of compound 13-e (483 mg, 1.14 mmol, 1 eq) in MeOH (5 mL) was added sodium methoxide (1 M, 5.69 mL, 5 eq). The reaction mixture was stirred at 50°C for 16 hours, then added with 1 M dilute hydrochloric acid to adjust the pH to around 5, and extracted with EA (30 mL × 2). The combined organic phases were washed with saturated brine (30 mL × 2), dried over anhydrous sodium sulfate, filtered, and concentrated to obtain compound 13-f.

Step G: To a solution of compound 13-f (117 mg, 309.22 µmol, 1 eq) in 1,4-dioxane (2 mL) was added hydrochloric acid (4 M, 2 mL, 25.87 eq). The reaction mixture was stirred at 60°C for 16 hours, then was added with 2 M sodium hydroxide aqueous solution to adjust the pH to around 8, and filtered. The filter cake was added with MTBE (10 mL), stirred for 1 hour, and filtered. The filter cake was dried to obtain compound 13. ¹H NMR (DMSO-*d₆*, 400 MHz): δ ppm 9.54 (br s, 1H), 7.51 - 7.41 (m, 1H), 7.32 - 7.23 (m, 1H), 7.12 (br t, *J* = 8.0Hz, 1H), 6.95 (br d, *J=* 6.1 Hz, 1H), 4.81 - 4.68 (m, 1H), 4.39 (s, 1H), 1.90 - 1.80 (m, 4H), 1.77 - 1.63 (m, 4H), 1.53 - 1.42 (d, *J=* 6.7 Hz, 3H); LCMS(ESI) m/z: 321.1(M+1).

### Example 14

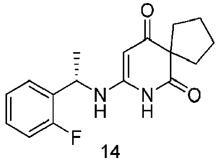

### Synthetic route:

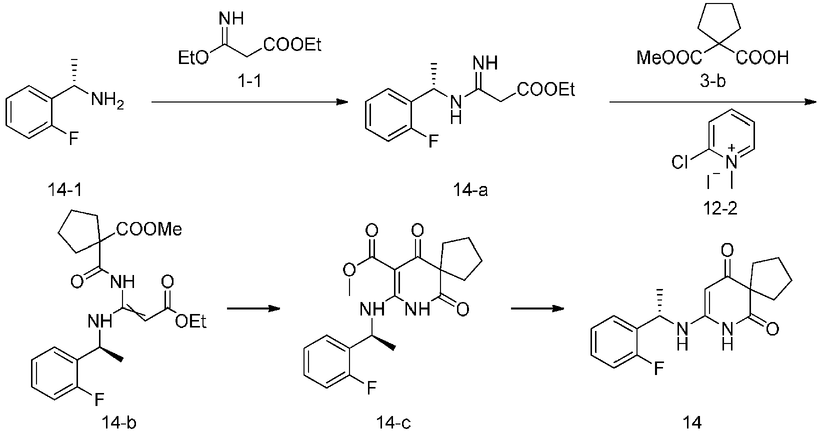

Step A: At 20°C, to a solution of compound 14-1 (0.8 g, 5.75 mmol, 1 eq) in EtOH (10 mL) was added compound 1-1 (915.04 mg, 5.75 mmol, 1 eq) and DIEA (1.49 g, 11.50 mmol, 2.00 mL, 2 eq). The reaction mixture was stirred at 25°C for 16 hours, then concentrated to obtain compound 14-a.

Step B: To a solution of compound 14-a (1 g, 3.48 mmol, purity of 87.68%, 1 eq) in THF (10 mL) was added DIEA (673.77 mg, 5.21 mmol, 908.04 µL, 1.5 eq), then compound 12-2 (1.33 g, 5.21 mmol, 1.5 eq) and compound 3-b (598.40 mg, 3.48 mmol, 1 eq) were added thereto. The reaction mixture was stirred at 25°C for 0.5 hours, then concentrated. The residue was added with water (50 mL), and extracted with EA (20 mL × 3). The combined organic phases were washed with saturated brine (20 mL × 2), dried over anhydrous sodium sulfate, filtered, and concentrated. The residue was purified by column chromatography (PE: EtOAc = 10:1) to obtain compound 14-b.

Step C: Under nitrogen atmosphere, to a solution of compound 14-b (231 mg, 568.34 µmol, 1 eq) in MeOH (5 mL) was added sodium methoxide (1 M, 2.84 mL, 5 eq). The reaction mixture was stirred at 50°C for 16 hours, then added with 1 M dilute hydrochloric acid to adjust the pH to about 5, diluted with water (20 mL), and extracted with EA (10 mL × 3). The combined organic phases were washed with saturated brine (10 mL × 2), dried over anhydrous sodium sulfate, filtered, and concentrated to obtain compound 14-c.

Step D: To a solution of compound 14-c (170 mg, 433.99 µmol, purity of 92%, 1 eq) in 1,4-dioxane (8.29 mL) was added hydrochloric acid (4 M, 8.29 mL, 76.4 eq). The reaction mixture was stirred at 50°C for 16 hours, added with 1 M sodium hydroxide aqueous solution to adjust the pH to around 9, and then extracted with EA (5 mL × 4). The combined organic phases were washed with saturated brine (10 mL × 2), dried over anhydrous sodium sulfate, filtered, and concentrated. The residue was added with MTBE (5 mL), stirred for 2 hours, and filtered. The filter cake was dried to obtain compound 14. ¹H NMR (DMSO-*d₆*, 400 MHz): δ ppm 9.70 (br s, 1H), 7.45-7.39 (m, 1H), 7.39-7.32 (m, 1H), 7.25 - 7.20 (m, 2H), 7.15 (br s, 1H), 4.81 - 4.73 (m, 1H), 4.38 (s, 1H), 1.89 - 1.79 (m, 4H), 1.74 - 1.64 (m, 4H), 1.48 (d, *J=* 6.8 Hz, 3H); LCMS(ESI) m/z: 303.2(M+1).

**Example 15**

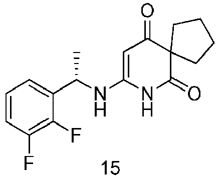

### Synthetic route:

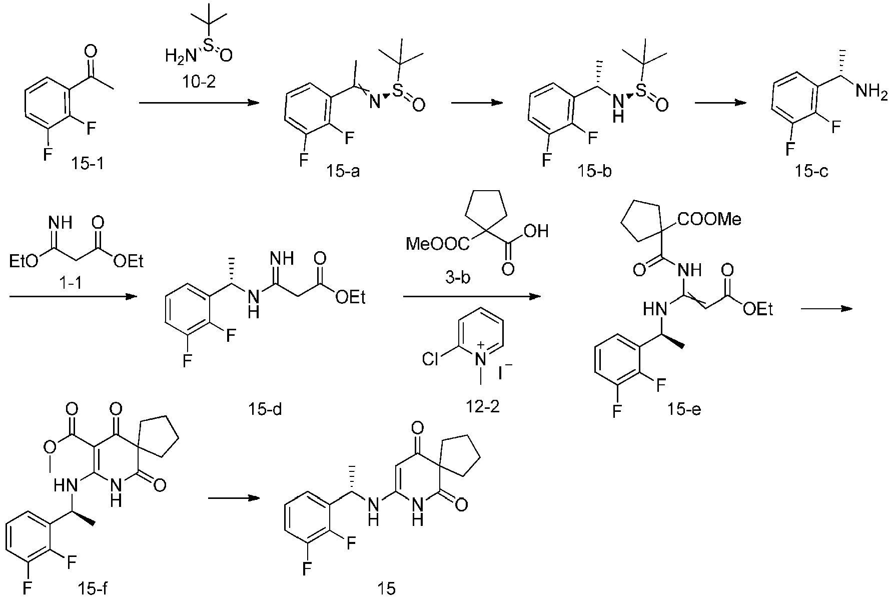

Step A: At 20°C, to a solution of compound 15-1 (1 g, 6.37 mmol, purity of 99.53%, 1 eq) in THF (10 mL) was added compound 10-2 (927.16 mg, 7.64 mmol, 1.2 eq) and titanium(IV) ethoxide (4.36 g, 19.11 mmol, 3.97 mL, 3 eq). The reaction mixture was stirred at 50°C for 16 hours, added with compound 10-2 (386.02 mg, 3.19 mmol, 0.5 eq), and stirred at 50°C for another 1.5 hours. After cooling to 0°C, the reaction mixture was diluted with ethyl acetate (30 mL), slowly added with water (20 mL), stirred for 0.5 hours, and filtered. The filtrate was washed with saturated brine (10 mL × 2), dried over anhydrous sodium sulfate, filtered, and concentrated to obtain compound 15-a.

Step B: Under nitrogen atmosphere at -78°C, to a solution of compound 15-a (950 mg, 3.66 mmol, 1 eq) in THF (10 mL) was slowly added L-selectride (1 M, 4.40 mL, 1.2 eq). The reaction mixture was stirred at -78°C for 2 hours, then at 0°C was slowly added to a saturated ammonium chloride aqueous solution (15 mL). The mixture was diluted with water (30 mL), extracted with ethyl acetate (10 mL × 3). The combined organic phases were washed with saturated brine (15 mL × 2), dried over anhydrous sodium sulfate, filtered, and concentrated. The residue was purified by column chromatography (PE: EtOAc = 5:1) to obtain compound 15-b.

Step C: At 20°C, to a solution of compound 15-b (1.15 g, 4.39 mmol, 1 eq) in MeOH (10 mL) was added HCl/MeOH (4M, 1.10 mL, 1 eq). The reaction mixture was stirred at 20°C for 16 hours, and concentrated to obtain the hydrochloride of compound 15-c.

Step D: Under nitrogen atmosphere at 20°C, to a solution of the hydrochloride of compound 15-c (0.887 g) in EtOH (10 mL) was added compound 1-1 (898.41 mg, 4.59 mmol, 8.14e⁻¹ eq, HCl) and DIEA (2.92 g, 22.58 mmol, 3.93 mL, 4 eq). The reaction mixture was stirred at 20°C for 16 hours, and concentrated to obtain compound 15-d.

Step E: Under nitrogen atmosphere, to a solution of compound 15-d (2.3 g, 8.51 mmol, 1 eq) in THF (25 mL) was added compound 3-b (1.47 g, 8.51 mmol, 1 eq), DIEA (1.65 g, 12.76 mmol, 2.22 mL, 1.5 eq), and compound 12-2 (3.26 g, 12.76 mmol, 1.5 eq). The reaction mixture was stirred at 20°C for 32 hours, then concentrated. The residue was diluted with water (50 mL), and extracted with EA (10 mL × 4). The combined organic phases were washed with saturated brine (15 mL × 2), dried over anhydrous sodium sulfate, filtered, and concentrated. The residue was purified by column chromatography (PE: EtOAc = 20:1) to obtain compound 15-e.

Step F: Under nitrogen atmosphere, to a solution of compound 15-e (260 mg, 612.58 µmol, 1 eq) in MeOH (3 mL) was added sodium methoxide (1 M, 3 mL, 4.90 eq). The reaction mixture was stirred at 50°C for 16 hours, added with 1 M dilute hydrochloric acid to adjust the pH to around 5, added with water (10 mL), and extracted with EA (5 mL × 4). The combined organic phases were washed with saturated brine (10 mL × 2), dried over anhydrous sodium sulfate, filtered, and concentrated. The residue was purified by thin-layer chromatography (PE: EtOAc = 20:1) to obtain compound 15-f.

Step G: To a solution of compound 15-f (60 mg, 158.58 µmol, 1 eq) in 1,4-dioxane (2 mL) was added hydrochloric acid (4 M, 2 mL, 50.45 eq). The reaction mixture was stirred at 60°C for 19 hours, then was added with 1 M sodium hydroxide aqueous solution to adjust the pH to around 9, and filtered. The filter cake was added with MTBE (2 mL), stirred for 2 hours, and filtered. The filter cake was dried to obtain compound 15. ¹H NMR (DMSO-*d₆*, 400 MHz): δ ppm 9.55 (m, 1H), 7.41 - 7.33 (m, 1H), 7.28 - 7.20 (m, 2H), 7.01 (br d, *J=* 4.6 Hz, 1H), 4.83 (br s, 1H), 4.40 (s, 1H), 1.91 - 1.79 (m, 4H), 1.75 - 1.65 (m, 4H), 1.50 (d, *J=* 6.8 Hz, 3H); LCMS(ESI) m/z: 321.2(M+1).

### Example 16

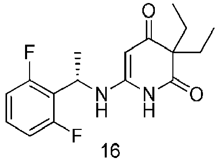

### Synthetic route:

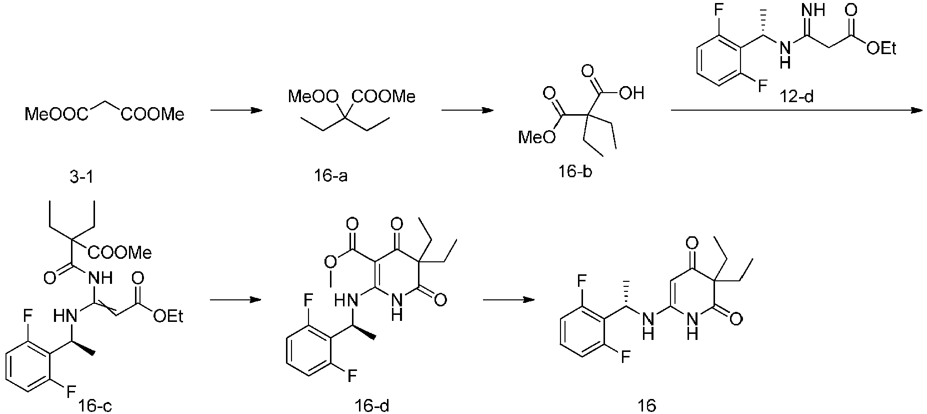

Step A: Under nitrogen atmosphere, to a solution of compound 3-1 (5 g, 37.85 mmol, 4.35 mL, 1 eq) and iodoethane (12.99 g, 83.26 mmol, 6.66 mL, 2.2 eq) in DMF (50 mL) was added cesium carbonate (27.13 g, 83.26 mmol, 2.2 eq). The reaction mixture was stirred at 20°C for 16 hours, added with water (200 mL), and extracted with EA (200 mL). The organic phase was washed with saturated brine, dried over anhydrous sodium sulfate, filtered, and concentrated to obtain compound 16-a.

Step B: To a solution of compound 16-a (7 g, 37.19 mmol, 1 eq) in MeOH (40 mL) and water (40 mL) was added sodium hydroxide (1.64 g, 40.91 mmol, 1.1 eq). The reaction mixture was stirred at 20°C for 16 hours, then concentrated. The residue was added with water (100 mL), then extracted with MTBE (100 mL). After separating the phases, the aqueous phase was added with 1 M dilute hydrochloric acid to adjust the pH to around 5, and then extracted with EA (100 mL). The organic phase was washed with saturated brine (100 mL), dried over anhydrous sodium sulfate, filtered, and concentrated to obtain compound 16-b.

Step C: Under nitrogen atmosphere at 0°C, to a solution of compound 12-d (1.2 g, 4.44 mmol, 1 eq) and compound 16-b (928.09 mg, 5.33 mmol, 1.2 eq) in ACN (20 mL) was added N-methylimidazole (1.82 g, 22.20 mmol, 1.77 mL, 5 eq) and TCFH (2.49 g, 8.88 mmol, 2 eq). The reaction mixture was stirred at 0°C for 1 hour, then concentrated. The residue was purified by silica gel column chromatography (PE: EtOAc = 1:0 to 10:1) to obtain compound 16-c.

Step D: Under nitrogen atmosphere, to a solution of compound 16-c (1.55 g, 3.63 mmol, 1 eq) in MeOH (20 mL) was added sodium tert-butoxide (1.75 g, 18.17 mmol, 5 eq). The reaction mixture was stirred at 20°C for 16 hours. The reaction mixture was added to 1 N dilute hydrochloric acid (20 mL), and concentrated. The residue was added with water (30 mL), and extracted with EA (30 mL). The organic phase was washed with saturated brine (30 mL), dried over anhydrous sodium sulfate, filtered, and concentrated. The residue was purified by silica gel column chromatography (PE: EtOAc = 10:1 to 3:1) to obtain compound 16-d.

Step E: To a solution of compound 16-d (0.15 g, 394.34 µmol, 1 eq) in 1,4-dioxane (5 mL) was added hydrochloric acid (4 M, 5 mL, 50.72 eq). The reaction mixture was stirred at 50°C for 16 hours, then was added with 1 N sodium hydroxide to adjust the pH to around 7, and concentrated to get rid of 1,4-dioxane. The residue was added with MTBE (20 mL), and filtered. The filter cake was dried under high vacuum to obtain compound 16. ¹H NMR (DMSO-*d₆*, 400 MHz): δ ppm 9.69 (br s, 1H), 7.45 - 7.37 (m, 1H), 7.14 (t, *J=* 8.5 Hz, 2H), 6.94 (br d, *J* = 7.6 Hz, 1H), 4.88 (br t, *J* = 6.8 Hz, 1H), 4.58 (s, 1H), 1.76 - 1.50 (m, 7H), 0.65 (t, *J=* 7.3 Hz, 3H), 0.48 (t, *J=* 7.3 Hz, 3H); LCMS(ESI) m/z: 323.4(M+1).

### Example 17

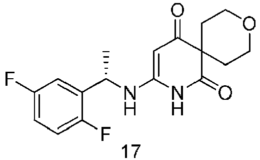

### Synthetic route:

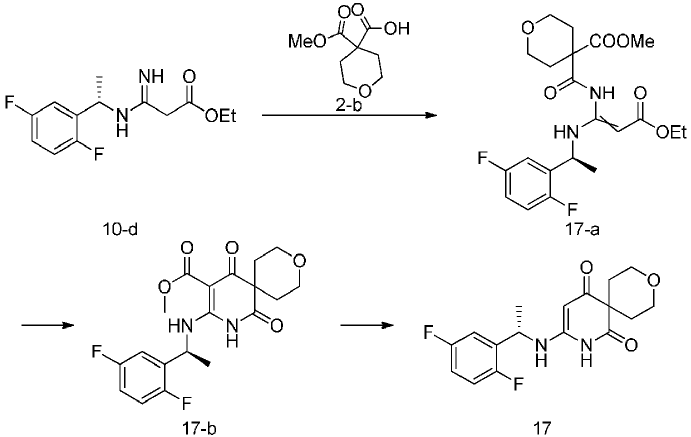

Step A: Under nitrogen atmosphere at 0°C, to a solution of compound 2-b (325.84 mg, 1.73 mmol, 1.3 eq) and compound 10-d (0.36 g, 1.33 mmol, 1 eq) in ACN (10 mL) was added N-methylimidazole (546.80 mg, 6.66 mmol, 530.88 µL, 5 eq) and TCFH (747.45 mg, 2.66 mmol, 2 eq). The reaction mixture was stirred at 20°C for 2 hours, then concentrated. The residue was purified by silica gel column chromatography (PE: EtOAc = 10:1 to 5:1) to obtain compound 17-a.

Step B: Under nitrogen atmosphere, to a solution of compound 17-a (0.34 g, 771.96 µmol, 1 eq) in MeOH (5 mL) was added sodium tert-butoxide (370.94 mg, 3.86 mmol, 5 eq). The reaction mixture was stirred at 20°C for 16 hours, added with 1 N dilute hydrochloric acid to adjust the pH to around 5, and concentrated. The residue was added with water (10 mL), and extracted with EA (10 mL). The organic phase was washed with saturated brine (10 mL), dried over anhydrous sodium sulfate, filtered, and concentrated. The residue was purified by silica gel column chromatography (PE: EtOAc = 10:1 to 2:1) to obtain compound 17-b.

Step C: Under nitrogen atmosphere, to a solution of compound 17-b (0.15 g, 380.35 µmol, 1 eq) in 1,4-dioxane (5 mL) was added hydrochloric acid (4 M, 5 mL, 52.58 eq). The reaction mixture was stirred at 50°C for 20 hours, then was added with saturated sodium hydroxide aqueous solution to adjust the pH to around 7, and concentrated to get rid of 1,4-dioxane. The residue was added with water (10 mL) and MTBE (20 mL), stirred for 30 minutes, and filtered. The filter cake was dried under high vacuum to obtain compound 17. ¹H NMR (DMSO-*d*₆, 400 MHz): δ ppm 9.67 (br s, 1H), 7.34 - 7.25 (m, 2H), 7.20 (dt, *J* = 4.1, 8.1 Hz, 1H), 7.07 (br d, *J* = 5.9 Hz, 1H), 4.76 (br s, 1H), 4.38 (s, 1H), 3.79 - 3.69 (m, 4H), 1.91 - 1.77 (m, 2H), 1.67 - 1.52 (m, 2H), 1.47 (d, *J=* 6.8 Hz, 3H); LCMS(ESI) m/z: 337.3(M+1).

### Example 18

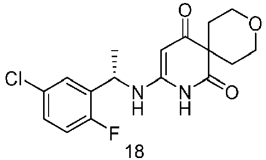

### Synthetic route:

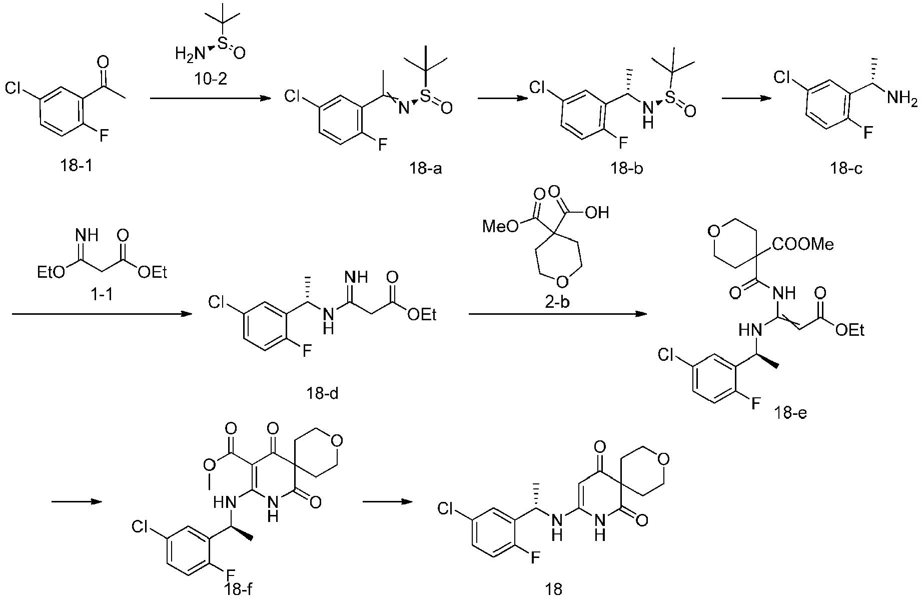

Step A: At 20°C, to a solution of compound 18-1 (6.89 g, 39.92 mmol, 1 eq) in THF (70 mL) was added compound 10-2 (5.81 g, 47.91 mmol, 1.2 eq) and titanium(IV) ethoxide (27.32 g, 119.77 mmol, 24.84 mL, 3 eq). The reaction mixture was stirred at 60°C for 16 hours, and ethyl acetate (100 mL) was added thereto. After cooling to 0°C, the mixture was slowly added with water (20 mL), stirred for 0.5 hours, and filtered. The filtrate was washed with saturated brine (50 mL × 3), dried over anhydrous sodium sulfate, filtered, and concentrated to obtain compound 18-a.

Step B: Under nitrogen atmosphere at -78°C, to a solution of compound 18-a (7.1 g, 25.75 mmol, 1 eq) in THF (100 mL) was slowly added dropwise L-selectridee (1 M, 25.75 mL, 1 eq). The reaction mixture was slowly warmed to 0°C, stirred for 1 hour, added with 0.5 N dilute hydrochloric acid (100 mL), and extracted with ethyl acetate (100 mL). The organic phase was washed with saturated brine (100 mL), dried over anhydrous sodium sulfate, filtered, and concentrated. The residue was purified by column chromatography (PE: EtOAc = 10:1 to 3:1) to obtain compound 18-b.

Step C: To compound 18-b (2 g, 7.20 mmol, 1 eq) was added a solution of HCl/MeOH (4 M, 25 mL, 13.89 eq). The reaction mixture was stirred at 50°C for 1 hour, and concentrated to obtain the hydrochloride of compound 18-c.

Step D: Under nitrogen atmosphere at 20°C, to a solution of the hydrochloride of compound 18-c (2 g) in EtOH (30 mL) was added compound 1-1 (2.05g, 10.47 mmol, 1.1 eq, HCl) and DIEA (3.69 g, 28.56 mmol, 4.97 mL, 3 eq). The reaction mixture was stirred at 20°C for 16 hours, and concentrated. The residue was added with water (50 mL), added with acetic acid to adjust the pH to around 5, and extracted with EA (50 mL). After separating the phases, the aqueous phase was added with saturated sodium bicarbonate solution to adjust the pH to around 9, then extracted with EA (50 mL × 2). The combined organic phases were washed with saturated brine (50 mL), dried over anhydrous sodium sulfate, filtered, and concentrated to obtain compound 18-d.

Step E: Under nitrogen atmosphere at 0°C, to a solution of compound 2-b (1.02 g, 5.44 mmol, 1.3 eq) and compound 18-d (1.2 g, 4.19 mmol, 1 eq) in ACN (10 mL) was added N-methylimidazole (1.72 g, 20.93 mmol, 1.67 mL, 5 eq) and TCFH (2.35 g, 8.37 mmol, 2 eq). The reaction mixture was stirred at 20°C for 1 hour, then concentrated. The residue was purified by silica gel column chromatography (PE: EtOAc = 10:1 to 5:1) to obtain compound 18-e.

Step F: Under nitrogen atmosphere, to a solution of compound 18-e (1.2 g, 2.63 mmol, 1 eq) in MeOH (10 mL) was added sodium tert-butoxide (1.01 g, 10.51 mmol, 4 eq). The reaction mixture was stirred at 50°C for 1 hour, added with 1 N dilute hydrochloric acid to adjust the pH to around 5, and concentrated. The residue was added with water (10 mL), and extracted with EA (10 mL). The organic phase was washed with saturated brine (10 mL), dried over anhydrous sodium sulfate, filtered, and concentrated. The residue was purified by silica gel column chromatography (PE: EtOAc = 10:1 to 2:1) to obtain compound 18-f.

Step G: Under nitrogen atmosphere, to a solution of compound 18-f (0.69 g, 1.68 mmol, 1 eq) in 1,4-dioxane (15 mL) was added hydrochloric acid (4 M, 15 mL, 35.72 eq). The reaction mixture was stirred at 50°C for 16 hours, then added with 1 N sodium hydroxide aqueous solution to adjust the pH to around 7, and concentrated to get rid of 1,4-dioxane. The residue was added with water (50 mL) and MTBE (50 mL), stirred for 30 minutes, and filtered. The filter cake was dried under high vacuum to obtain compound 18. ¹H NMR (DMSO-*d₆*, 400 MHz): δ ppm 9.63 (br s, 1H), 7.54 - 7.49 (m, 1H), 7.45 - 7.39 (m, 1H), 7.34 - 7.27 (m, 1H), 6.98 (br s, 1H), 4.77 (br s, 1H), 4.40 (s, 1H), 3.79 - 3.69 (m, 4H), 1.90 - 1.78 (m, 2H), 1.59 (br dd, *J* = 14.3, 19.1 Hz, 2H), 1.47 (d, *J* = 6.7 Hz, 3H); LCMS(ESI) m/z: 353.3(M+1).

### Bioactivity testing:

### Experiment example 1: Experiment on inhibitory effect of cardiac myosin

### ATPase activity.

### Experimental reagents:

Cardiac tropomyosin/troponin complex (Cytoskeleton, Cat. # TT05)
Cardiac myosin S1 (Cytoskeleton, Cat. # MYS03)
Cardiac actin (Cytoskeleton, Cat. # AD99-A)
ATPase assay kit with biochemical reagents (Cytoskeleton, Cat. # BK051)

### Experimental steps:

### 1) Preparing the compounds

a) The compounds were diluted 4-fold with DMSO in Echo, with eight concentration gradients. 200 nL of each compound was transferred into a 96-well plate (Corning-3696) respectively.
b) The plate was centrifuged at 1000 rpm for 15 seconds and then sealed for later use.

### 2) Preparing F-actin

a) A buffer with 5 mM Pipes-KOH (pH 7.0), 500 µM ATP, and 500 µM dithiothreitol was prepared, and 1 mg of F-actin was dissolved in 2.5 mL of the buffer, resulting in a protein concentration of 0.4 mg/mL.
b) The mixture was left to stand at room temperature for 10 minutes, allowing the protein to be fully dissolved.
c) 2.0 mM MgCl₂ and 2.0 mM EGTA were added thereto. The mixture was left to stand at room temperature for 20 minutes to form a protein polymer.

### 3) Preparing thin filaments

a) 200 µL of ice water was added to dissolve 1 mg of cardiac tropomyosin/troponin complex, resulting in a protein concentration of 5 mg/mL.
b) The mixture was added with 1000 µL of the F-actin prepared in step 1, and uniformly mixed.
c) The mixture was left to stand at room temperature for 20 minutes.
d) The mixture was centrifuged at 87K x g at 4°C for 1.5 hours.
e) A PM12 buffer with 12 mM Pipes-KOH (pH 7.0) and 2 mM MgCl₂ was prepared, and 1200 µL of the PM12 buffer was added to re-suspend the protein.

### 4) Preparing a reaction mixture and initiating the experiment

a) 250 µL of ice-cold PM12 buffer was added to 250 µg of S1 myosin, resulting in a protein concentration of 1 mg/mL.
b) Reagents were added in the following sequence and mixed to obtain the reaction mixture:
   400 µL of PM12;
   400 µL of 5x MSEG (from the ATPase assay kit with biochemical reagents);
   1200 µL of actin/cardiac tropomyosin/troponin complex;
   40 µL of myosin S1;
   40 µL of 100x PNP (from the ATPase assay kit with biochemical reagents);
   10.4 µL of 100 mM ATP.
c) 10 µL of 440 µM CaCl₂ solution was added to the 96-well plate. The plate was placed in a 37°C incubator to preheat.
d) 100 µL of the reaction mixture was added to the 96-well plate. The plate was centrifuged at 1000 rpm for 10 seconds.
e) Readings were continuously recorded for 10 minutes with a 30-second interval using a SpectraMax 340PC at a temperature of 37°C, and at a wavelength of 360 nm.

### Data analysis:

Prism was employed for data analysis. The experimental results are shown in Table 1.

**Table 1: Test results of IC₅₀ values indicating inhibitory effect of compounds of present disclosure on cardiac myosin ATPase activity**

| **Compound** | **IC₅₀ (µM)** |
|---|---|
| 1 | 14 |
| 2 | 3.97 |
| 3 | 0.73 |
| 4 | 2.2 |
| 5 | 2.08 |
| 6 | 1.71 |
| 7 | 0.3 |
| 9 | 0.04 |
| 10 | 0.22 |
| 11 | 0.51 |
| 12 | 0.06 |
| 13 | 1.08 |
| 14 | 1.19 |
| 15 | 0.21 |

**Conclusion:** The compounds of the present disclosure exhibit good inhibitory activity against cardiac myosin ATPase.

### Experimental example 2: In vivo pharmacokinetic evaluation in rats

### Experimental purpose:

To evaluate pharmacokinetic parameters of the compounds of the present disclosure in rats in vivo.

### Experimental design:

1) Experimental drugs: compounds of the present disclosure;
2) Experimental animals: Four male SD rats, aged 7 to 9 weeks, randomly divided into 2 groups, with 2 rats in each group;
3) Drug preparation: An appropriate amount of the drug was weighed and dissolved in a mixed solvent of DMAC, PEG-400, and 30% 2-HP-β-CD in a ratio of 5:25:70, resulting in a concentration of 0.2 mg/mL.

### Experimental operations:

Animals in group 1 were administered the drug at a dose of 0.2 mg/kg at a concentration of 0.2 mg/mL by single injection through the tail vein. Animals in group 2 were administered the compound at a dose of 1 mg/kg at a concentration of 0.2 mg/mL by intragastric gavage. Plasma samples were collected from the animals at 0.0833 (tail vein injection group only), 0.25, 0.5, 1, 2, 4, 6, 8, and 24 hours after administration.

### Data analysis:

The drug concentrations in the plasma samples were determined using the LC-MS/MS method to obtain pharmacokinetic test results for the tested drugs. The results are shown in Table 2.

**Table 2: Pharmacokinetic test results of compounds of present disclosure**

| | | | | | | |
|---|---|---|---|---|---|---|
| Compound 3 | Tail Vein Injection Group | Clearance Rate | Initial Concentration | Volume of Distribution | Half-life | Area Under Curve |
| | | Cl (mL/Kg/min) | C₀ (nM) | Vd(L/Kg) | T_{1/2} (h) | AUC (nM•h) |
| | | 8.11 | 773 | 1.6 | 2.31 | 1331 |
| | Intragastric Administration Group | Peak Concentration | Peak Concentration Time | Area Under Curve | Bioavailability | -- |
| | | Cₘₐₓ (nM) | T_{1/2} (h) | AUC (nM•h) | F (%) | -- |
| | | 1385 | 4.81 | 7316 | 110 | -- |
| Compound 7 | Tail Vein Injection Group | Clearance rate | Initial Concentration | Volume of Distribution | Half-life | Area Under Curve |
| | | Cl (mL/Kg/min) | C₀ (nM) | Vd (L/Kg) | T_{1/2} (h) | AUC (nM•h) |
| | | 2.93 | 3695 | 0.885 | 3.86 | 3533 |
| | Intragastric Administration Group | Peak Concentration | Peak Concentration Time | Area Under Curve | Bioavailability | -- |
| | | Cₘₐₓ (nM) | T_{1/2} (h) | AUC (nM•h) | F (%) | -- |
| | | 2140 | 5.96 | 14802 | 88.1 | -- |
| Compound 9 | Tail Vein Injection Group | Clearance rate | Initial Concentration | Volume of Distribution | Half-life | Area Under Curve |
| | | Cl (mL/Kg/min) | C₀ (nM) | Vd (L/Kg) | T_{1/2} (h) | AUC (nM•h) |
| | | 4.78 | 4818 | 1.73 | 5.93 | 2114 |
| | Intragastric Administration Group | Peak Concentration | Peak Concentration Time | Area Under Curve | Bioavailability | -- |
| | | Cₘₐₓ (nM) | T_{1/2} (h) | AUC (nM•h) | F (%) | -- |
| | | 1945 | 7.35 | 8110 | 76.7 | -- |
| Compound 10 | Tail Vein Injection Group | Clearance rate | Initial Concentration | Volume of Distribution | Half-life | Area Under |
| | | | | | | Curve |
| | | Cl (mL/Kg/min) | C₀ (nM) | Vd (L/Kg) | T_{1/2} (h) | AUC (nM•h) |
| | | 6.0 | 1460 | 2.7 | 6.37 | 1294 |
| | Intragastric Administration Group | Peak Concentration | Peak Concentration Time | Area Under Curve | Bioavailability | -- |
| | | Cₘₐₓ (nM) | T_{1/2} (h) | AUC (nM•h) | F (%) | -- |
| | | 1307 | 4.83 | 7929 | 123 | -- |

| | | | | | | |
|---|---|---|---|---|---|---|
| -- indicates non-existence. | | | | | | |

**Conclusion:** The compounds of the present disclosure exhibit good in vivo pharmacokinetic properties in rats.

## Claims

1. A compound of formula (I) or a pharmaceutically acceptable salt thereof, 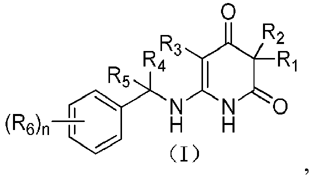 wherein
Ri and R₂ are each independently selected from H, F, Cl, Br, I, -OH, -NH₂, -CN, C₁₋₄ alkyl, and C₁₋₄ alkoxy, wherein the C₁₋₄ alkyl and C₁₋₄ alkoxy are each independently and optionally substituted by 1, 2, or 3 Rₐ;
alternatively, R₁ and R₂ together with the carbon atom to which they are attached form a C₃₋₆ cycloalkyl ring or a 3- to 6-membered heterocycloalkyl ring, wherein the C₃₋₆ cycloalkyl ring and the 3- to 6-membered heterocycloalkyl ring are each independently and optionally substituted by 1, 2, 3, or 4 R_{b};
R₃ is selected from H and F;
R₄ is selected from H, C₁₋₄ alkyl, and C₃₋₄ cycloalkyl, wherein the C₁₋₄ alkyl and C₃₋₄ cycloalkyl are each independently and optionally substituted by 1, 2, or 3 R_{c};
R₅ is selected from H and C₁₋₄ alkyl;
each R₆ is independently selected from H, F, Cl, Br, I, -OH, -NH₂, -CN, C₁₋₄ alkyl, and C₁₋₄ alkoxy, wherein the C₁₋₄ alkyl and C₁₋₄ alkoxy are each independently and optionally substituted by 1, 2, or 3 R_{d};
each Rₐ is independently selected from F, Cl, Br, I, -OH, -NH₂, -CN, C₁₋₄ alkyl, C₁₋₄ alkoxy, -CORₐ₁, -CO₂Rₐ₁, -SO₂Rₐ₁, -SO₂NRₐ₁Rₐ₂, and -CONRₐ₁Rₐ₂, wherein the C₁₋₄ alkyl and C₁₋₄ alkoxy are each independently and optionally substituted by 1, 2, or 3 R;
Rₐ₁ and Rₐ₂ are each independently selected from H and C₁₋₄ alkyl;
alternatively, Rₐ₁ and Rₐ₂ together with the nitrogen atom to which they are attached form a 4- to 6-membered heterocycloalkyl ring, wherein the 4- to 6-membered heterocycloalkyl ring is independently and optionally substituted by 1, 2, 3, or 4 Rₑ;
each R_{b} is independently selected from F, Cl, Br, I, -OH, -NH₂, -CN, C₁₋₄ alkyl, C₁₋₄ alkoxy, -COR_{b1}, -CO₂R_{b1}, -SO₂R_{b1}, -SO₂NR_{b1}R_{b2}, and -CONR_{b1}R_{b2}, wherein the C₁₋₄ alkyl and the Ci-4 alkoxy are each independently and optionally substituted by 1, 2, or 3 R;
R_{b1} and R_{b2} are each independently selected from H and C₁₋₄ alkyl;
alternatively, R_{b1} and R_{b2} together with the nitrogen atom to which they are attached form a 4- to 6-membered heterocycloalkyl ring, wherein the 4- to 6-membered heterocycloalkyl ring is independently and optionally substituted by 1, 2, 3, or 4 R_{f};
each R_{c} is independently selected from F, Cl, Br, I, -OH, -NH₂, -CN, C₁₋₄ alkyl, and C₁₋₄ alkoxy;
each R_{d} is independently selected from F, Cl, Br, I, -OH, -NH₂, -CN, C₁₋₄ alkyl, and C₁₋₄ alkoxy;
each Rₑ is independently selected from F, Cl, Br, I, -OH, -NH₂, -CN, C₁₋₄ alkyl, and C₁₋₄ alkoxy;
each R_{f} is independently selected from F, Cl, Br, I, -OH, -NH₂, -CN, C₁₋₄ alkyl, and C₁₋₄ alkoxy;
each R is independently selected from F, Cl, Br, I, -OH, -NH₂, and -CN;
n is selected from 1, 2, 3, or 4;
the 3- to 6-membered heterocycloalkyl ring and the 4- to 6-membered heterocycloalkyl ring each independently comprise 1, 2, 3, or 4 atoms or atomic groups each independently selected from N, O, S, and NH.

2. The compound or the pharmaceutically acceptable salt thereof according to claim 1, wherein Rₐ, R_{c}, R_{d}, Rₑ, and R_{f} are each independently selected from F and Cl.

3. The compound or the pharmaceutically acceptable salt thereof according to claim 1, wherein R₁ and R₂ are each independently selected from -CH₃ and -CH₂CH₃, wherein the -CH₃ and -CH₂CH₃ are each independently and optionally substituted by 1, 2, or 3 Rₐ.

4. The compound or the pharmaceutically acceptable salt thereof according to claim 2, wherein R₁ and R₂ are each independently selected from -CH₃ and -CH₂CH₃.

5. The compound or the pharmaceutically acceptable salt thereof according to claim 1, wherein R_{b1} and R_{b2} are each independently selected from -CH₃ and -CH₂CH₃.

6. The compound or the pharmaceutically acceptable salt thereof according to claim 1 or 5, wherein each R_{b} is independently selected from F, Cl, Br, -OCH₃, -COCH₃, -CO₂CH₃, and - CO₂CH₂CH₃.

7. The compound or the pharmaceutically acceptable salt thereof according to claim 1, wherein R₁ and R₂ together with the carbon atom to which they are attached form 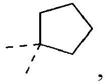 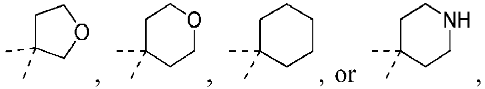 wherein the 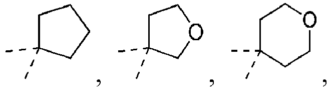 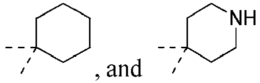 are each independently and optionally substituted by 1, 2, 3, or 4 R_{b}.

8. The compound or the pharmaceutically acceptable salt thereof according to claim 7, wherein R₁ and R₂ together with the carbon atom to which they are attached form

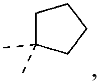

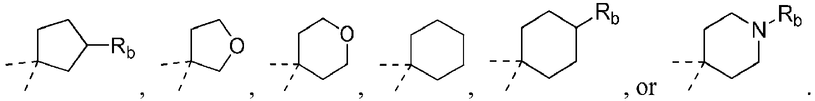

9. The compound or the pharmaceutically acceptable salt thereof according to claim 8, wherein R₁ and R₂ together with the carbon atom to which they are attached form

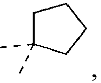

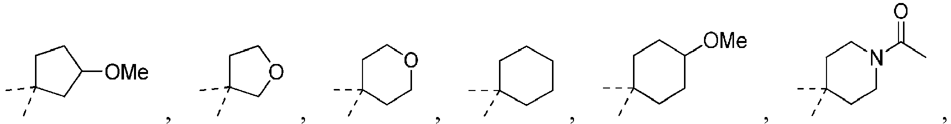

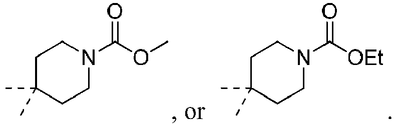

10. The compound or the pharmaceutically acceptable salt thereof according to claim 1, wherein the structural moiety 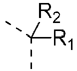 is selected from

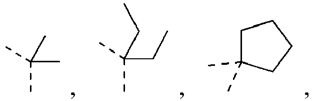

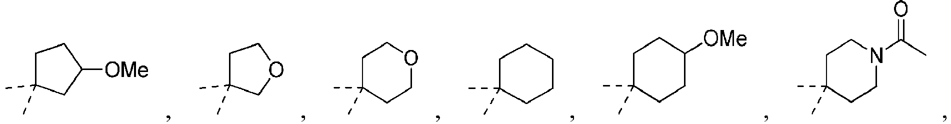

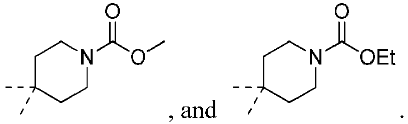

11. The compound or the pharmaceutically acceptable salt thereof according to claim 1, wherein R₃ is selected from H.

12. The compound or the pharmaceutically acceptable salt thereof according to claim 1, wherein R₄ is selected from -CH₃ and -CH₂CH₃.

13. The compound or the pharmaceutically acceptable salt thereof according to claim 1, wherein R₅ is selected from H.

14. The compound or the pharmaceutically acceptable salt thereof according to claim 1, wherein each R₆ is independently selected from H, F, Cl, and -CH₃, wherein the -CH₃ is optionally substituted by 1, 2, or 3 R_{d}.

15. The compound or the pharmaceutically acceptable salt thereof according to claim 14, wherein each R₆ is independently selected from H, F, Cl, and -CH₃.

16. The compound or the pharmaceutically acceptable salt thereof according to claim 1, wherein the compound has a structure of formula (1-1): 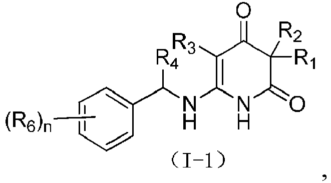 wherein n, R₁, R₂, R₃, R₄, and R₆ are as defined in claim 1.

17. The compound or the pharmaceutically acceptable salt thereof according to claim 16, wherein the compound has a structure of formula (I-1A) or formula (I-1B): 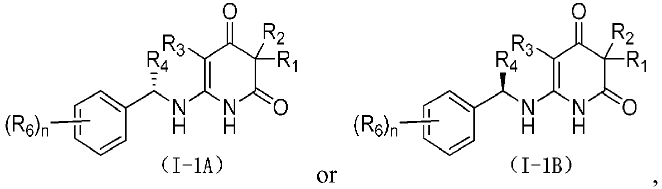 wherein n, R₁, R₂, R₃, R₄, and R₆ are as defined in claim 16, and R₄ is not H.

18. A compound of the following formula or a pharmaceutically acceptable salt thereof,

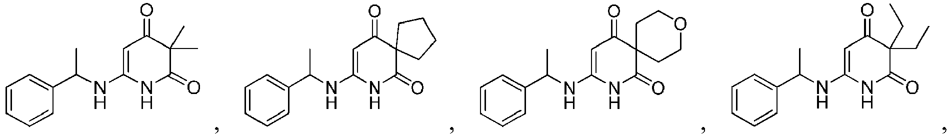

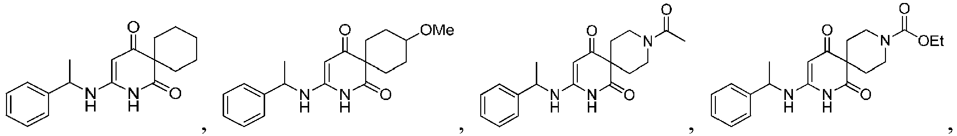

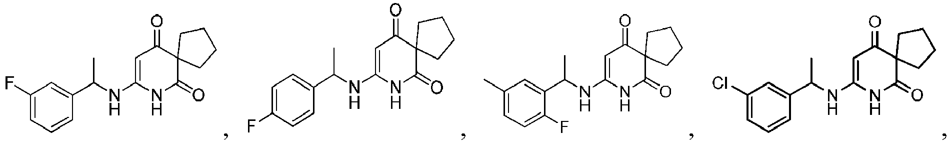

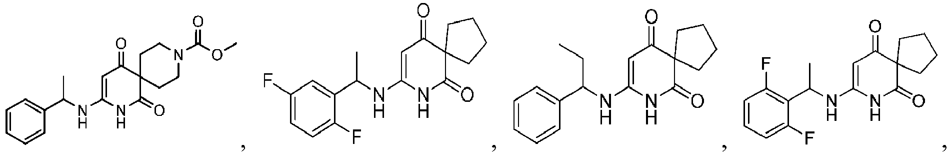

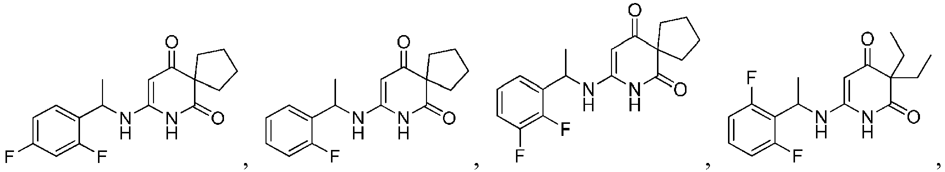

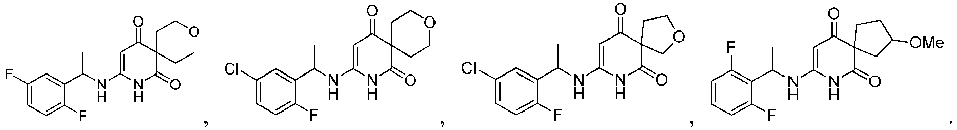

19. A compound of the following formula or a pharmaceutically acceptable salt thereof,

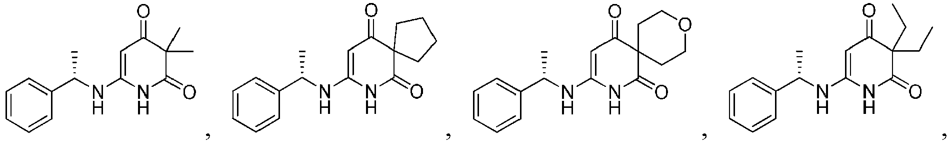

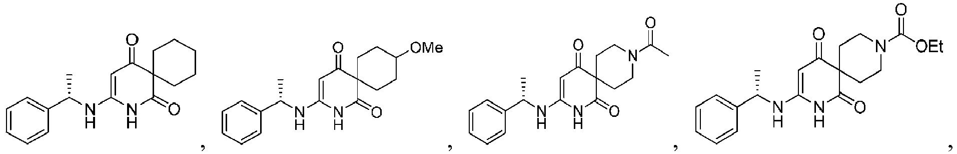

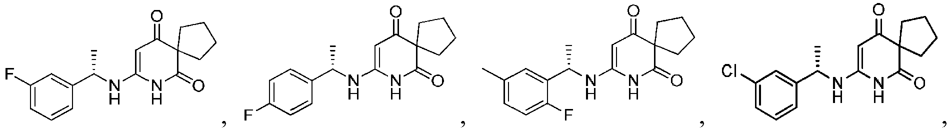

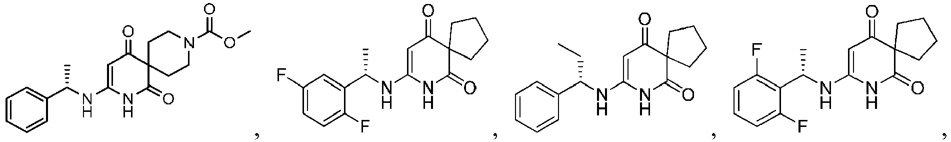

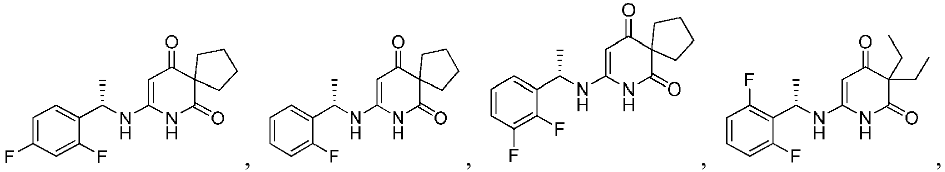

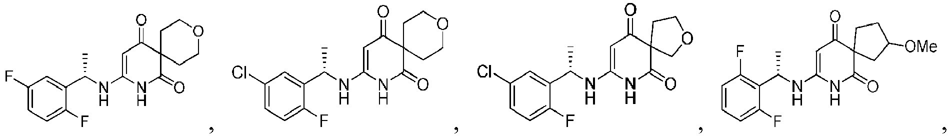

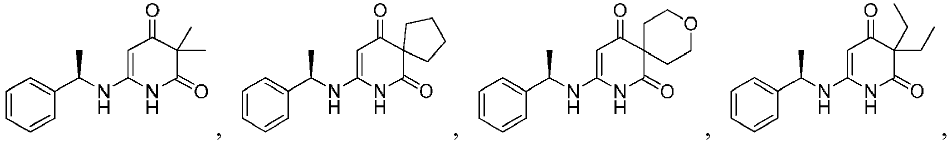

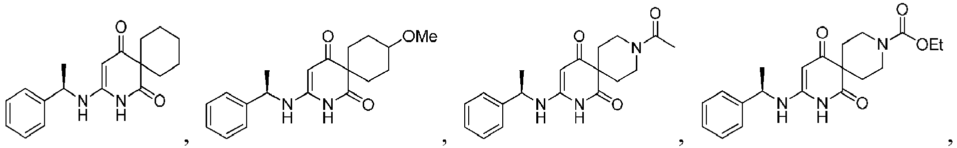

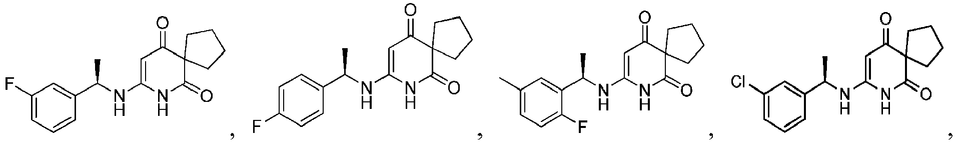

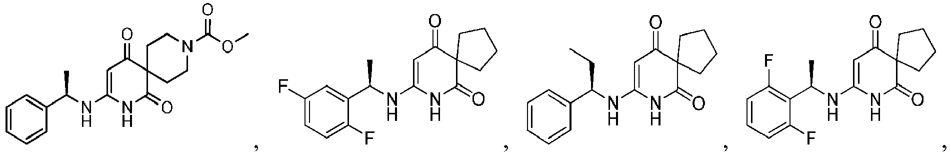

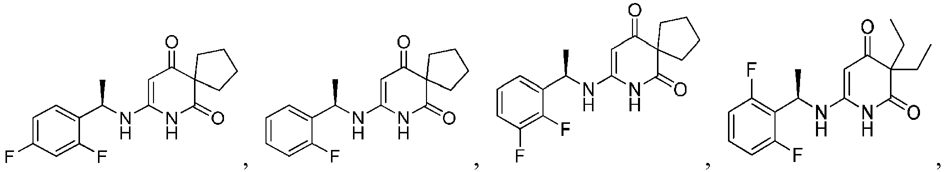

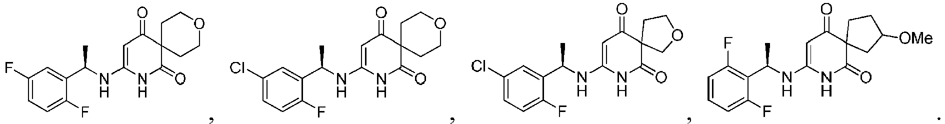

20. A pharmaceutical composition comprising a therapeutically effective amount of the compound or the pharmaceutically acceptable salt thereof according to any one of claims 1 to 19 and a pharmaceutically acceptable carrier.

21. A use of the compound or the pharmaceutically acceptable salt thereof according to any one of claims 1 to 19, or the pharmaceutical composition according to claim 20 in the manufacture of a cardiac myosin inhibitor medicament.

22. A use of the compound or the pharmaceutically acceptable salt thereof according to any one of claims 1 to 19, or the pharmaceutical composition according to claim 20 in the manufacture of a medicament for treating heart failure and hypertrophic cardiomyopathy.
